(19)

Europäisches
Patentamt
European
Patent Office
Office européen
des brevets

(11) **EP 2 623 512 A1**

(12) **EUROPEAN PATENT APPLICATION**
published in accordance with Art. 153(4) EPC

(43) Date of publication:
**07.08.2013  Bulletin 2013/32**

(21) Application number: **11828953.7**

(22) Date of filing: **22.09.2011**

(51) Int Cl.:
*C07K 7/06* (2006.01)       *A61K 38/00* (2006.01)
*A61P 9/14* (2006.01)        *A61P 13/12* (2006.01)
*A61P 19/02* (2006.01)       *A61P 29/00* (2006.01)
*C07K 19/00* (2006.01)       *C12N 15/09* (2006.01)

(86) International application number:
**PCT/JP2011/071671**

(87) International publication number:
**WO 2012/043397 (05.04.2012 Gazette 2012/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **01.10.2010   JP 2010224211**

(71) Applicant: **MMT CO., LTD**
**Osaka-shi, Osaka 540-0008 (JP)**

(72) Inventors:
• **MASAKI, Osamu**
  **Osaka-shi**
  **Osaka 540-0008 (JP)**

• **TOMITA, Tetsuya**
  **Toyonaka-shi**
  **Osaka 561-0801 (JP)**

(74) Representative: **Schnappauf, Georg**
**Dr. Volker Vossius**
**Patent- und Rechtsanwaltskanzlei**
**Geibelstrasse 6**
**81679 München (DE)**

(54)   **PEPTIDE CAPABLE OF BINDING TO IMMUNOGLOBULIN**

(57)    The present invention relates to a peptide capable of binding to an immunoglobulin, and to a fusion protein containing such a peptide. The present invention also relates to a pharmaceutical composition for the treatment or prevention of a disease caused by binding between Clq and an immunoglobulin, which comprises a peptide capable of binding to the immunoglobulin or a fusion protein with such a peptide, and to the like. The pharmaceutical composition provided by the present invention is effective particularly in treating arthritis and rheumatism.

EP 2 623 512 A1

Sequence Listing

SEQUENCE LISTING

<110> MMT CO., LTD.

<120> Peptide having ability of binding to immunoglobulin

<130> 38-144

<140> EP 11 828 953.7
<141> 2011-09-22

<150> JP 2010-224211
<151> 2010-10-01

<160> 22

<170> PatentIn version 3.2

<210> 1
<211> 6
<212> PRT
<213> Artificial Sequence

<220>
<223> Immunoglobulin binding peptide

<400> 1

Pro Gly Leu Tyr Tyr Phe
1               5


<210> 2
<211> 6
<212> PRT
<213> Artificial Sequence

<220>
<223> Immunoglobulin binding peptide

<400> 2

Ala Gly Leu Tyr Tyr Phe
1               5


<210> 3
<211> 6
<212> PRT
<213> Artificial Sequence

<220>
<223> Immunoglobulin binding peptide

<400> 3

Pro Ala Leu Tyr Tyr Phe
1               5


<210> 4
<211> 6
<212> PRT
<213> Artificial Sequence

```
<220>
<223>   Immunoglobulin binding peptide

<400>   4

Pro Gly Ala Tyr Tyr Phe
1               5


<210>   5
<211>   6
<212>   PRT
<213>   Artificial Sequence

<220>
<223>   Immunoglobulin binding peptide

<400>   5

Pro Gly Leu Ala Tyr Phe
1               5


<210>   6
<211>   6
<212>   PRT
<213>   Artificial Sequence

<220>
<223>   Immunoglobulin binding peptide

<400>   6

Pro Gly Leu Tyr Ala Phe
1               5


<210>   7
<211>   6
<212>   PRT
<213>   Artificial Sequence

<220>
<223>   Immunoglobulin binding peptide

<400>   7

Pro Gly Leu Tyr Tyr Ala
1               5


<210>   8
<211>   15
<212>   PRT
<213>   Artificial Sequence

<220>
<223>   Immunoglobulin binding peptide

<400>   8

Ser Thr Gly Lys Phe Thr Cys Lys Val Pro Gly Leu Tyr Tyr Phe
1               5                   10                  15
```

```
<210>  9
<211>  6
<212>  PRT
<213>  Artificial Sequence

<220>
<223>  Immunoglobulin binding peptide

<400>  9

Pro Gly Leu Asp Lys Phe
1               5


<210>  10
<211>  6
<212>  PRT
<213>  Artificial Sequence

<220>
<223>  Immunoglobulin binding peptide

<400>  10

Pro Gly Leu Asp Arg Phe
1               5


<210>  11
<211>  6
<212>  PRT
<213>  Artificial Sequence

<220>
<223>  Immunoglobulin binding peptide

<400>  11

Pro Gly Leu Asp His Phe
1               5


<210>  12
<211>  6
<212>  PRT
<213>  Artificial Sequence

<220>
<223>  Immunoglobulin binding peptide

<400>  12

Pro Gly Leu Glu Lys Phe
1               5


<210>  13
<211>  6
<212>  PRT
<213>  Artificial Sequence

<220>
<223>  Immunoglobulin binding peptide

<400>  13
```

Pro Gly Leu Glu Arg Phe
1               5


<210>  14
<211>  6
<212>  PRT
<213>  Artificial Sequence

<220>
<223>  Immunoglobulin binding peptide

<400>  14

Pro Gly Leu Glu His Phe
1               5


<210>  15
<211>  6
<212>  PRT
<213>  Artificial Sequence

<220>
<223>  Immunoglobulin binding peptide

<400>  15

Pro Gly Leu Ser Ser Phe
1               5


<210>  16
<211>  18
<212>  DNA
<213>  Artificial Sequence

<220>
<223>  Nucleic acid encoding immunoglobulin binding peptide

<400>  16
cccggtctcg ataaattc                                                        18


<210>  17
<211>  18
<212>  DNA
<213>  Artificial Sequence

<220>
<223>  Nucleic acid encoding immunoglobulin binding peptide

<400>  17
cccggtctcg atcgtttc                                                        18


<210>  18
<211>  18
<212>  DNA
<213>  Artificial Sequence

<220>
<223>  Nucleic acid encoding immunoglobulin binding peptide

```
<400>  18
cccggtctcg atcatttc                                                    18


<210>  19
<211>  18
<212>  DNA
<213>  Artificial Sequence

<220>
<223>  Nucleic acid encoding immunoglobulin binding peptide

<400>  19
cccggtctcg aaaaattc                                                    18


<210>  20
<211>  18
<212>  DNA
<213>  Artificial Sequence

<220>
<223>  Nucleic acid encoding immunoglobulin binding peptide

<400>  20
cccggtctcg aacgtttc                                                    18


<210>  21
<211>  18
<212>  DNA
<213>  Artificial Sequence

<220>
<223>  Nucleic acid encoding immunoglobulin binding peptide

<400>  21
cccggtctcg aacatttc                                                    18


<210>  22
<211>  18
<212>  DNA
<213>  Artificial Sequence

<220>
<223>  Nucleic acid encoding immunoglobulin binding peptide

<400>  22
cccggtctct cttctttc                                                    18
```

**Description**

Technical Field

The present invention relates to a peptide capable of binding to an immunoglobulin, and to a fusion protein containing such a peptide. The present invention also relates to a pharmaceutical composition for the treatment or prevention of a disease caused by binding between Clq and an immunoglobulin, which comprises a peptide capable of binding to the immunoglobulin or a fusion protein with such a peptide, and to the like.

Background Art

C1q is a complement protein, which is known to act in the pathway of complement activation. For example, it has been considered that the activation of the classical pathway is caused by binding of C1q to the Fc region of an immunoglobulin molecule.

There are many types of diseases caused by binding between C1q and an immunoglobulin, of which typical examples include immune-complex diseases, such as systemic lupus erythematosus (SLE), glomerulonephritis, vasculitis, and arthritis; and rheumatism. It has been reported that patients with rheumatoid arthritis (RA) have high levels of C1q in their blood and will suffer from joint destruction in the future (Non-Patent Document 1). The above-described activation by C1q is suggested to be involved in these pathological conditions, and thus there is desire for development of an inhibitor of binding between C1q and an immunoglobulin molecule.

Currently, methotrexate and leflumide are often used as drugs for the treatment of rheumatoid arthritis; however, these drugs exhibit adverse effects and cannot be administered in large amounts.

Prior Art Document

Non-Patent Document

Non-Patent Document 1: Ochi T et al., Arthritis Rheum. 1988 Jan; 31(1): 37-43

Summary of the Invention

Problems to be Solved by the Invention

An object to be achieved by the present invention is to provide a peptide capable of binding to an immunoglobulin, a fusion protein containing such a peptide, and an effective and safe pharmaceutical composition for the treatment or prevention of a disease caused by binding between C1q and an immunoglobulin, which comprises a peptide capable of binding to the immunoglobulin or a fusion protein with such a peptide.

Means for Solving the Problems

The present inventors have found that peptides having C1q-derived amino acid sequences exhibit treatment and prevention effects in diseases caused by binding between C1q and immunoglobulins, such as rheumatoid arthritis (see, PCT International Publications Nos. WO 2009/025300 and WO 2010/084851). In addition, the present inventors have found that on the basis of results from extensive studies performed in order to increase peptide solubility in water, substitution of amino acids at specific positions in the peptides improves their water solubility and furthermore, does not cause a significant decrease in their biological activities, including immunoglobulin binding ability, and thus have completed the present invention.

Therefore, the present invention provides:

(1) A peptide capable of binding to an immunoglobulin having an amino acid sequence represented by:

$$\text{Pro-Gly-Leu-}X_4\text{-}X_5\text{- he} \qquad \text{(I)}$$

wherein $X_4$ and $X_5$ are not Tyr at the same time;

(2) The peptide according to (1), wherein either or both of $X_4$ and $X_5$ are a hydrophilic amino acid(s);

(3) The peptide according to (1), wherein both $X_4$ and $X_5$ are hydrophilic amino acids;

(4) The peptide according to (3), wherein each of $X_4$ and $X_5$ independently is an amino acid selected from the group consisting of Lys, Arg, His, Asp, Glu, Asn, Gln, Ser, and Thr;

(5) The peptide according to (1), which has the amino acid sequence depicted in any of SEQ ID NOs. 9 to 15;

(6) The peptide according to (1), which has the amino acid sequence depicted in SEQ ID NO. 12;

(7) The peptide according to any of (1) to (6), wherein one or more amino acids in the amino acid sequence represented by Formula (I), except for Gly, are substituted by the corresponding D-Amino acid(s);

(8) The peptide according to any of (1) to (6), wherein all of the amino acids in the amino acid sequence represented by Formula (I), except for Gly, are substituted by the corresponding D-amino acids;

(9) The peptide according to (8), which has an amino acid sequence of D-Pro-Gly-D-Leu-D-Glu-D-Lys-D-Phe;

(10) A nucleic acid encoding a peptide capable of binding to an immunoglobulin, wherein the nucleic acid encodes the peptide according to any of (1) to (6);

(11) The nucleic acid according to (10), wherein the nucleic acid encodes the peptide according to (5);

(12) The nucleic acid according to (10), wherein the nucleic acid encodes the peptide according to (6);

(13) A vector comprising the nucleic acid according to any of (10) to (12);

(14) A fusion protein in which the peptide according to any of (1) to (6) is added at the N- and/or C-terminus of a given protein;

(15) A fusion protein in which the peptide according to any of (7) to (9) is added at the N- and/or C-terminus of a given protein;

(16) A nucleic acid encoding the fusion protein according to (14);

(17) A vector comprising the nucleic acid according to (16);

(18) A cell comprising the nucleic acid according to any of (10) to (12), the nucleic acid according to (16), or the vector according to (13) or (17);

(19) A method for producing a peptide capable of binding to an immunoglobulin, which comprises the steps of:

(a) transforming a cell with the vector according to (13), and
(b) culturing the cell, thereby to produce the peptide;

(20) A peptide capable of binding to an immunoglobulin, which is obtainable by the method according to (19);

(21) A method for producing a fusion protein in which a peptide capable of binding to an immunoglobulin is added at the N- and/or C-terminus of a given protein, the method comprising the steps of:

(a) transforming a cell with the vector according to (17), and
(b) culturing the cell, thereby to produce the fusion protein;

(22) The method according to (21), wherein the method further comprises the step of removing the given protein from the fusion protein;

(23) A fusion protein obtainable by the method according to (21) or (22);

(24) A composition for allowing binding of an immunoglobulin, comprising the peptide according to any of (1) to (9) or the fusion protein according to (14) or (15);

(25) The composition according to (24) which is for determining the presence or amount of the immunoglobulin, or is for isolating the immunoglobulin;

(26) A means for allowing binding of an immunoglobulin, wherein the peptide according to any of (1) to (9) or the fusion protein according to (14) or (15) has been immobilized;

(27) The means according to (26) which is for determining the presence or amount of the immunoglobulin, or is for isolating the immunoglobulin;

(28) A method for allowing binding of an immunoglobulin, which comprises the steps of:

(a) adding to a sample the peptide according to any of (1) to (9) or the fusion protein according to (14) or (15), and
(b) determining a complex between the peptide or the fusion protein and the immunoglobulin;

(29) A kit for use in the method according to (28), comprising the peptide according to any of (1) to (9) or the fusion protein according to (14) or (15);

(30) The peptide according to any of (1) to (9) or the fusion protein according to (14) or (15) which has a label attached thereto;

(31) A method for detecting an antibody in a sample, which comprises reacting the labeled peptide or fusion protein according to (30) with the antibody in the sample, and then detecting the peptide or fusion protein bound to the antibody;

(32) A pharmaceutical composition for the treatment or prevention of a disease caused by binding between C1q and an immunoglobulin, comprising, as the active ingredient, the peptide according to any of (1) to (9), the fusion protein according to (14) or (15), the nucleic acid according to any of (10) to (12), the nucleic acid according to (16), or the vector according to (13) or (17);

(33) The pharmaceutical composition according to (32), wherein the disease is rheumatoid arthritis; and

(34) The pharmaceutical composition according to (32), wherein the disease is an immune-complex disease, such as systemic lupus erythematosus (SLE), glomerulonephritis, vasculitis, or arthritis.

Effects of the Invention

According the present invention, it is made possible to provide a peptide capable of binding to an immunoglobulin, a fusion protein containing such a peptide, a pharmaceutical composition which is more useful in the treatment or prevention of a disease caused by binding between C1q and an immunoglobulin, and others. It is also made possible to provide a pharmaceutical composition of which the virtual dosage amount is increased in comparison to conventional pharmaceutical compositions, by using in the above-described pharmaceutical composition the peptide of the present invention which has improved water solubility.

Brief Description of the Drawings

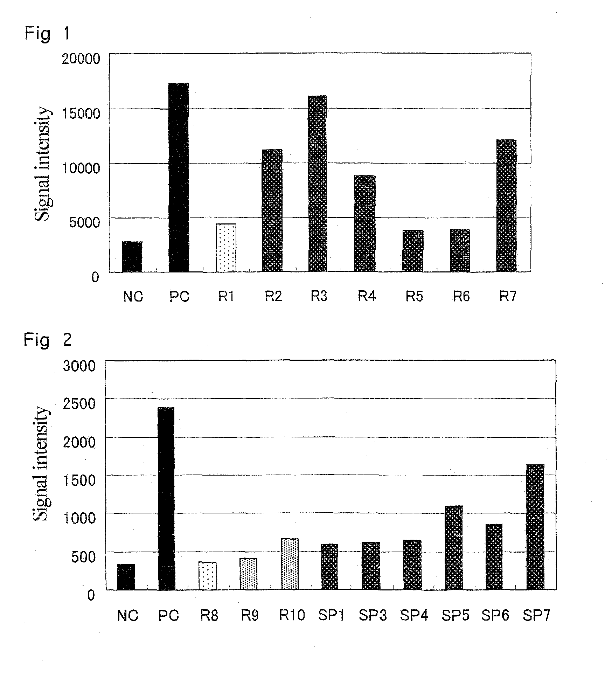

Fig. 1 shows that peptides R1 and R4 to R6 inhibit the binding between C1q and an immunoglobulin. In the figure, NC represents the result from samples in which peptide-free DMSO was added to reaction solutions which did not contain human immunoglobulin (IgG) labeled with alkaline phosphatase (ALP), and PC represents the result from samples in which peptide-free DMSO was added to reaction solutions which contained human immunoglobulin (IgG) labeled with alkaline phosphatase (ALP).

Fig. 2 shows that peptides SP1 to SP7 inhibit the binding between C1q and an immunoglobulin. In the figure, NC represents the result from samples in which peptide-free DMSO was added to reaction solutions which did not contain human immunoglobulin (IgG) labeled with alkaline phosphatase (ALP), and PC represents the result from samples in which peptide-free DMSO was added to reaction solutions which contained human immunoglobulin (IgG) labeled with alkaline phosphatase (ALP).

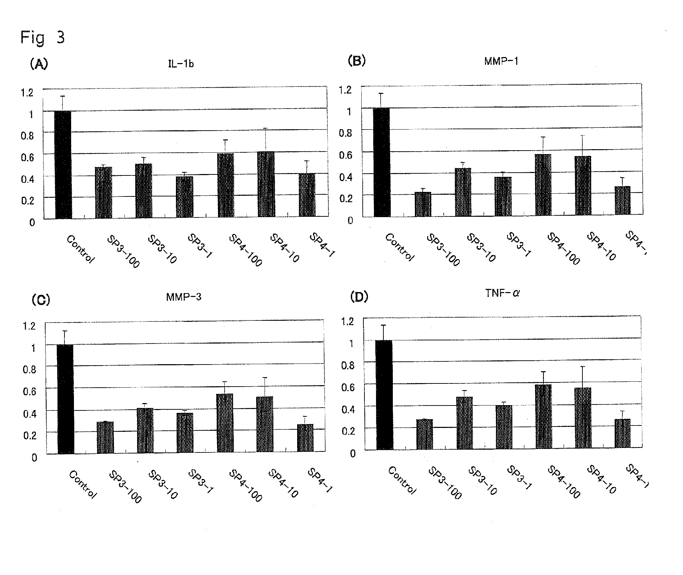

Fig. 3 shows that the production of each of IL-1$\beta$, TNF-$\alpha$, MMP-1, and MMP-3 induced by TNF-$\alpha$ stimulation of synovial cells, collected from a human RA patient was suppressed by the addition of the peptides of present invention. In each of the figures, Control represents the result from samples without added peptide; SP3-100, SP3-10, and SP3-1 represent the results from samples having peptide SP3 added thereto at 100 $\mu$g, 10 $\mu$g, and 1 $\mu$g, respectively; and SP4-100, SP4-10, and SP4-1 represent the results from samples having peptide SP4 added thereto at 100 $\mu$g, 10 $\mu$g, and 1 $\mu$g, respectively. ,

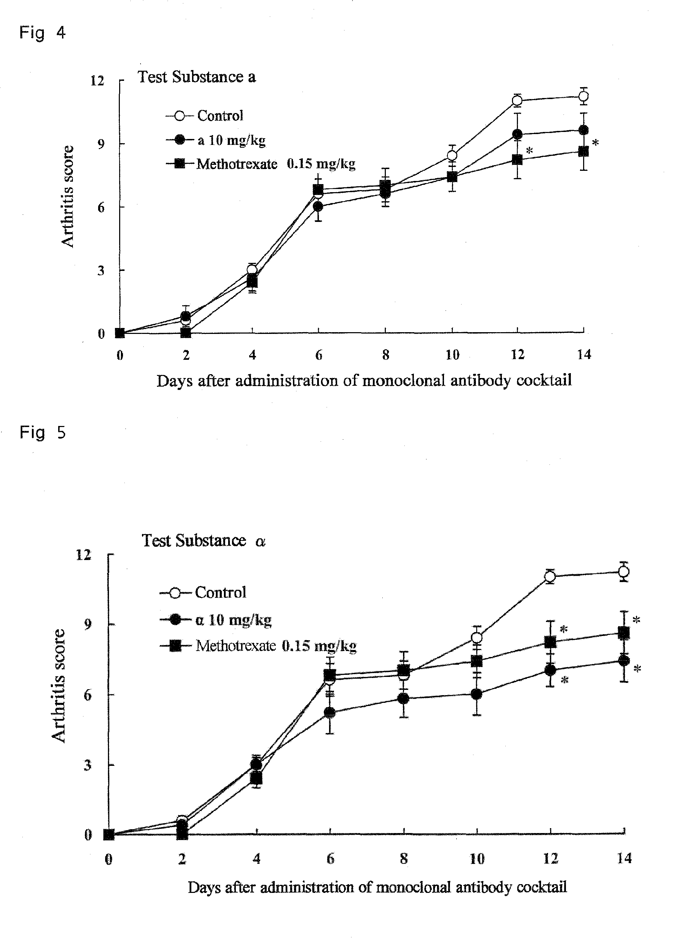

Fig. 4 is a graph showing, in terms of clinical score, an arthritis suppressing effect of test substance a using monoclonal antibody cocktail-induced arthritis mice. The open circles represent a control group, the filled circles represent a group receiving 10 mg/kg of test substance a, and the filled squares represent a group receiving 0.15 mg/kg of methotrexate.

Fig. 5 is a graph showing, in terms of clinical score, an arthritis suppressing effect of test substance $\alpha$ using monoclonal antibody cocktail-induced arthritis mice. The open circles represent a control group, the filled circles represent a group receiving 10 mg/kg of test substance $\alpha$, and the filled squares represent a group receiving 0.15 mg/kg of methotrexate.

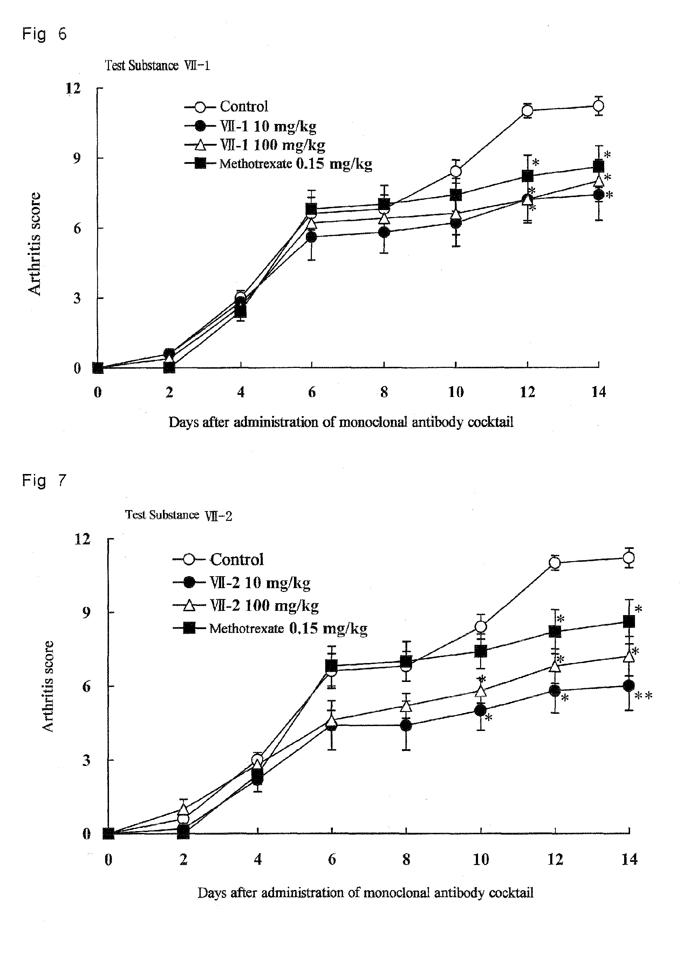

Fig. 6 is a graph showing, in terms of clinical score, an arthritis suppressing effect of test substance VII-1 using monoclonal antibody cocktail-induced arthritis mice. The open circles represent a control group, the filled circles represent a group receiving 10 mg/kg of test substance VII-1, the open triangles represent a group receiving 100 mg/kg of test substance VII-1, and the filled squares represent a group receiving 0.15 mg/kg of methotrexate.

Fig. 7 is a graph showing, in terms of clinical score, an arthritis suppressing effect of test substance VII-2 using monoclonal antibody cocktail-induced arthritis mice. The open circles represent a control group, the filled circles represent a group receiving 10 mg/kg of test substance VII-2, the open triangles represent a group receiving 100 mg/kg of test substance VII-2, and the filled squares represent a group receiving 0.15 mg/kg of methotrexate.

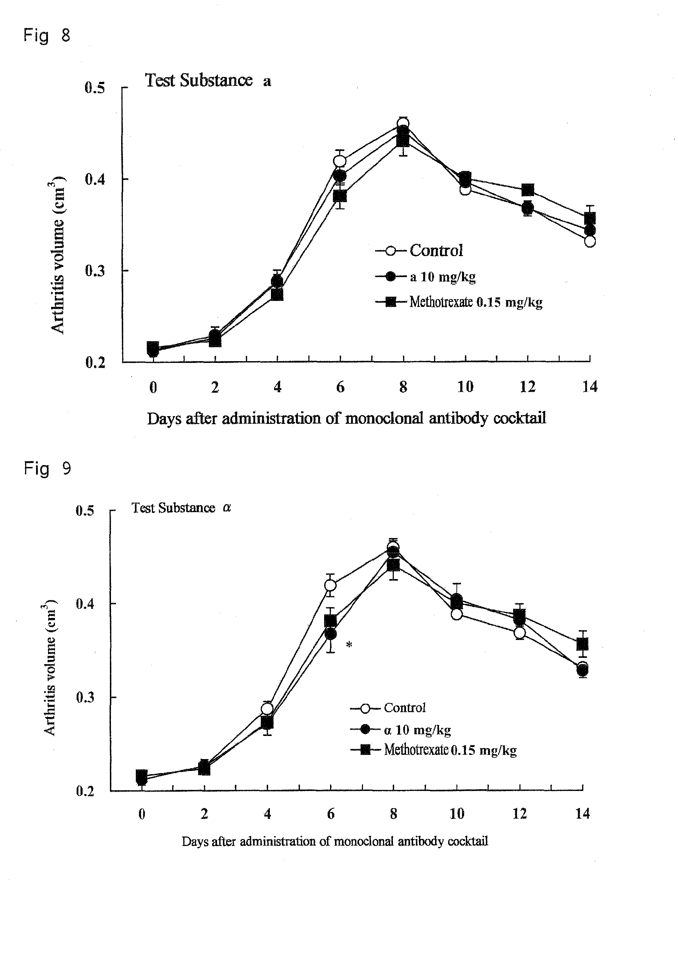

Fig. 8 is a graph showing, in terms of hind-limb volume, an arthritis suppressing effect of test substance a using monoclonal antibody cocktail-induced arthritis mice. The open circles represent a control group, the filled circles represent a group receiving 10 mg/kg of test substance a, and the filled squares represent a group receiving 0.15 mg/kg of methotrexate.

Fig. 9 is a graph showing, in terms of hind-limb volume, an arthritis suppressing effect of test substance $\alpha$ using monoclonal antibody cocktail-induced arthritis mice. The open circles represent a control group, the filled circles represent a group receiving 10 mg/kg of test substance $\alpha$, and the filled squares represent a group receiving 0.15 mg/kg of methotrexate.

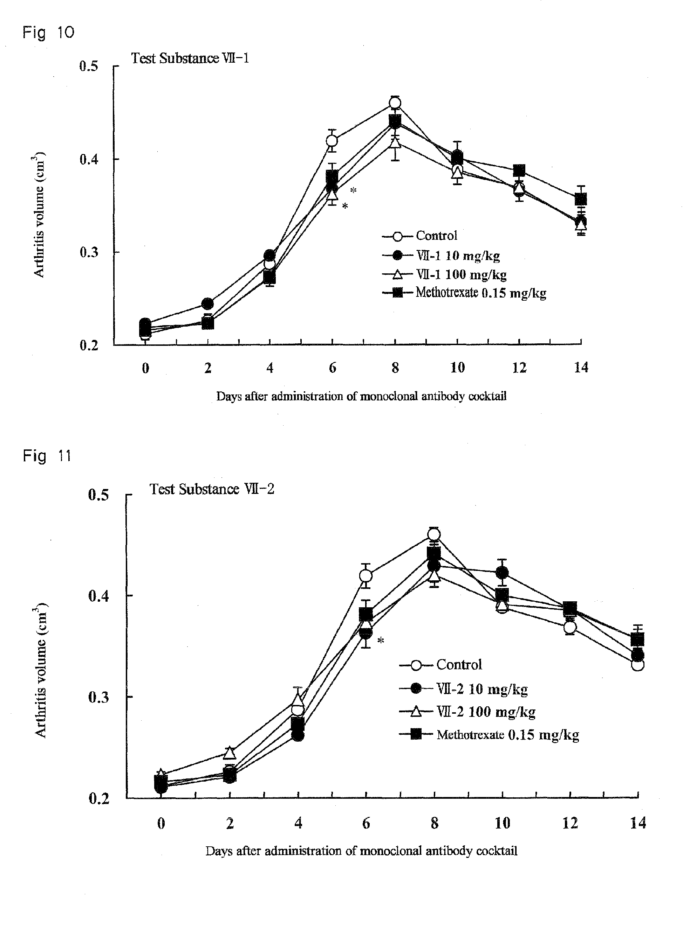

Fig. 10 is a graph showing, in terms of hind-limb volume, an arthritis suppressing effect of test substance VII-1 using monoclonal antibody cocktail-induced arthritis mice. The open circles represent a control group, the filled circles represent a group receiving 10 mg/kg of test substance VII-1, the open triangles represent a group receiving 100 mg/kg of test substance VII-1, and the filled squares represent a group receiving 0.15 mg/kg of methotrexate.

Fig. 11 is a graph showing, in terms of hind-limb volume, an arthritis suppressing effect of test substance VII-2 using monoclonal antibody cocktail-induced arthritis mice. The open circles represent a control group, the filled circles represent a group receiving 10 mg/kg of test substance VII-2, the open triangles represent a group receiving 100 mg/kg of test substance VII-2, and the filled squares represent a group receiving 0.15 mg/kg of methotrexate.

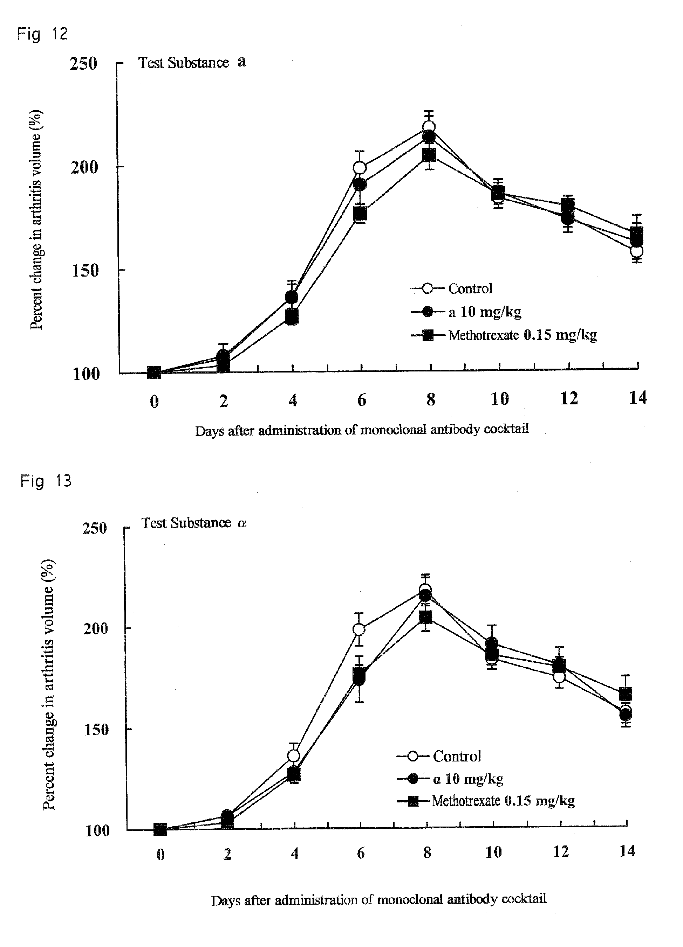

Fig. 12 is a graph showing, in terms of increasing rate of hind-limb volume, an arthritis suppressing effect of test substance a using monoclonal antibody cocktail-induced arthritis mice. The open circles represent a control group, the filled circles represent a group receiving 10 mg/kg of test substance a, and the filled squares represent a group receiving 0.15 mg/kg of methotrexate.

Fig. 13 is a graph showing, in terms of increasing rate of hind-limb volume, an arthritis suppressing effect of test substance α using monoclonal antibody cocktail-induced arthritis mice. The open circles represent a control group, the filled circles represent a group receiving 10 mg/kg of test substance α, and the filled squares represent a group receiving 0.15 mg/kg of methotrexate.

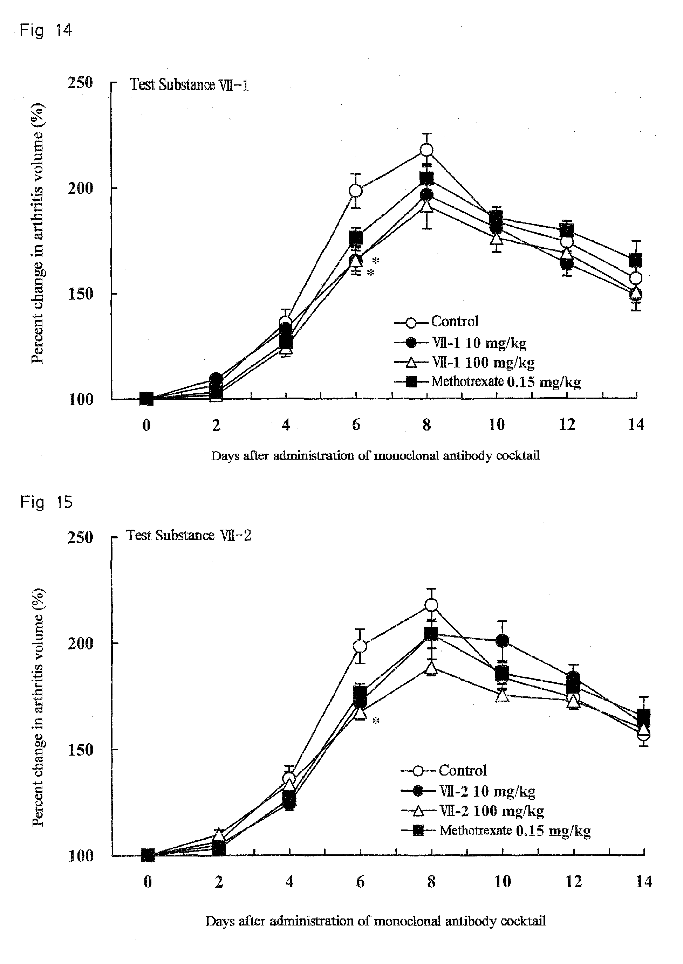

Fig. 14 is a graph showing, in terms of increasing rate of hind-limb volume, an arthritis suppressing effect of test substance VII-1 using monoclonal antibody cocktail-induced arthritis mice. The open circles represent a control group, the filled circles represent a group receiving 10 mg/kg of test substance VII-1, the open triangles represent a group receiving 100 mg/kg of test substance VII-1, and the filled squares represent a group receiving 0.15 mg/kg of methotrexate.

Fig. 15 is a graph showing, in terms of increasing rate of hind-limb volume, an arthritis suppressing effect of test substance VII-2 using monoclonal antibody cocktail-induced arthritis mice. The open circles represent a control group, the filled circles represent a group receiving 10 mg/kg of test substance VII-2, the open triangles represent a group receiving 100 mg/kg of test substance VII-2, and the filled squares represent a group receiving 0.15 mg/kg of methotrexate.

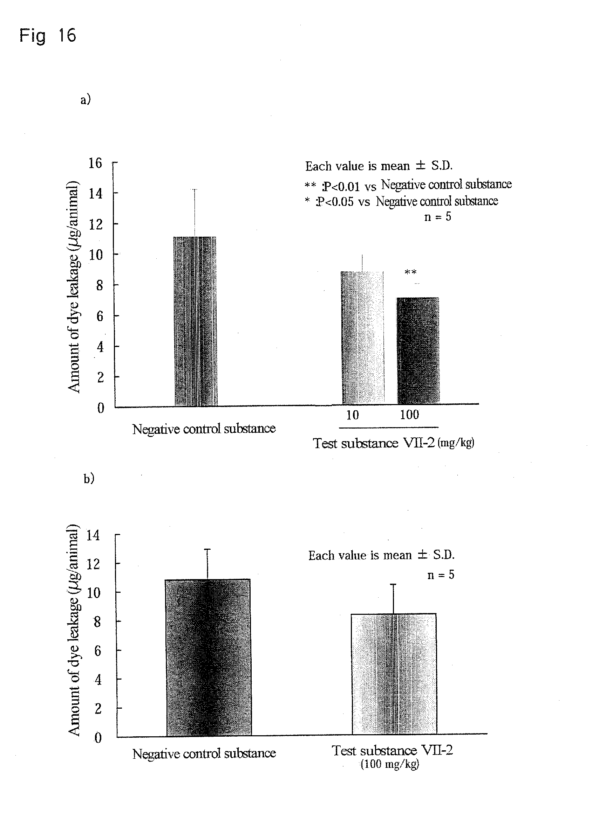

In Fig. 16, Fig. 16a is a graph showing effects of intraperitoneal administration of test substance VII-2 in Arthus reaction, and Fig. 16b is a graph showing effects of intravenous administration into tail vain of test substance VII-2 in Arthus reaction.

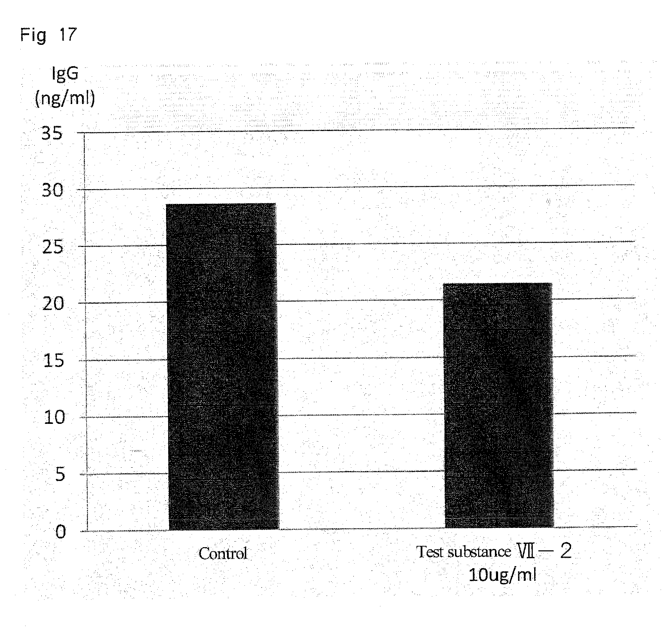

Fig. 17 is a graph showing an IgG production-suppressing effect of test substance VII-2 in a rheumatic joint model system in which synovial cells and B cells coexist.

Best Mode for Carrying Out the Invention

The present invention, in an aspect thereof, is directed to a peptide that has biological activities, such as immunoglobulin binding ability which is approximately equal to or higher than that of a peptide having the amino acid sequence (SEQ ID No: 1) represented by:

Pro-Gly-Leu-Tyr-Tyr-Phe            (a)

and that has improved water solubility. Biological activities of the peptide of the present invention can be examined by methods known in the art. For example, the immunoglobulin binding ability of a peptide may be determined by incubating the peptide in the presence of an immunoglobulin and examining the amount of the peptide bound to the immunoglobulin. Peptides of the present invention are directed to one having an amino acid sequence in which one or more (for example, 2, 3, 4, or 5) amino acids are substituted in the amino acid sequence (a) depicted in SEQ ID No: 1. In the peptides of the present invention, an amino acid to be replaced may be any amino acid and preferably is the 4th and/or 5th amino acid, further preferably both the 4th and 5th amino acids, from the N-terminus of the above-described amino acid sequence (a).

In the specification, amino acids may be denoted, instead of their names, by their one- or three-letter codes, which are well known to those skilled in the art.

For a peptide of the present invention, any of the amino acids in the amino acid sequence (a) may be substituted by other amino acids, as long as the peptide has immunoglobulin binding ability and good water solubility. For example, substitutions may be made with amino acids making up natural proteins, such as glycine, alanine, valine, leucine, isoleucine, serine, threonine, aspartic acid, glutamic acid, asparagine, glutamine, lysine, arginine, cysteine, methionine, phenylalanine, tyrosine, tryptophan, histidine, or proline, or alternatively with amino acids not making up natural proteins, such as β-alanine, γ-aminobutyric acid (GABA), 5-aminolevulinic acid, 5-aminovaleric acid, homocysteine, ornithine, 5-hydroxytryptophan, 3,4-dihydroxyphenylalanine (dopa), triiodotyronine, thyroxine, homolysine, norleucine, creatine, desmosine, norvaline, or iodotyrosine.

One or more amino acids in above-described amino acid sequence (a) is(are) substituted by a hydrophilic amino acid(s) or by an amino acid(s) derivatized or modified, so that water solubility of a peptide having such a substituted amino acid sequence can be improved. Therefore, in a preferable aspect of the present invention, an amino acid(s) in the amino acid sequence of Formula (a) is(are) substituted by a hydrophilic amino acid(s) or by an amino acid(s) derivatized or modified such that the peptide has increased water solubility. Examples of hydrophilic amino acids include, but are not limited to, serine (Ser), threonine (Thr), aspartic acid (Asp), glutamic acid (Glu), asparagine (Asn), glutamine (Gln), lysine (Lys), arginine (Arg), or histidine (His).

In general, in cases of peptide pharmaceuticals, methods of administration by intravenous or subcutaneous injection are often employed in view of their easy degradation in the digestive tract. For this reason, peptides having low water solubility have difficulties in their clinical application. On the other hand, oral administration, even when selected, will cause a problem that these peptides are prone to degradation. Peptides of the present invention with improved water solubility have resolved the disadvantages as described above, and exert superior effects when administered by intravenous and subcutaneous injection. In addition, peptides of the present invention with improved water solubility have an advantage of being easily used as materials.

In a preferable aspect, the present invention is directed to a peptide having an amino acid sequence represented by:

$$\text{Pro-Gly-Leu-}X_4\text{-}X_5\text{-Phe} \qquad \text{(I)}$$

wherein $X_4$ and $X_5$ are not Tyr at the same time. According to this aspect, a peptide of the present invention has an amino acid sequence in which either or both of amino acid residues $X_4$ and $X_5$ in the amino acid sequence (I) are an amino acid residue(s) other than Tyr. Preferably, a peptide of the present invention has an amino acid sequence in which both of amino acid residues $X_4$ and $X_5$ in the amino acid sequence (I) are amino acid residues other than Tyr. Amino acids $X_4$ and $X_5$ other than Tyr are the above-mentioned amino acids, preferably hydrophilic amino acids, and further preferably hydrophilic amino acids such as serine (Ser), threonine (Thr), aspartic acid (Asp), glutamic acid (Glu), asparagine (Asn), glutamine (Gln), lysine (Lys), arginine (Arg), or histidine (His). Preferred as amino acids $X_4$ and $X_5$ other than Tyr may be amino acids derivatized or modified such that the peptide has increased water solubility. $X_4$ and $X_5$ may be amino acids of the same kind or of kinds different from each other. When having any of such amino acid sequences, the peptide of the present invention is capable of binding to an immunoglobulin and has a higher water solubility than that of a peptide having the amino acid sequence (a) of SEQ ID No: 1.

Preferable peptides of the present invention include one having the amino acid sequence depicted in any of SEQ ID NOs: 9 to 15. These peptides are also capable of binding to an immunoglobulin.

In addition, a peptide of the present invention may have an amino acid sequence in which one or more (for example, 2, 3, 4, 5, or 6) amino acids in the amino acid sequence (I) are further substituted by the corresponding D-amino acid(s).

In a preferable aspect of the present invention, all of the amino acids in the amino acid sequence of Formula (I) may be substituted by the corresponding D-amino acids. Herein, when having any of such 'amino acid sequences, the peptide of the present invention is capable of binding to an immunoglobulin.

By substitution of an amino acid(s) in the amino acid sequence of Formula (I) with the corresponding D-amino acid(s), the peptide of the present invention will be made less likely to experience its proteolytic degradation, so that the peptide will have an increased stability and a prolonged half-life within the body of a subject to whom the peptide has been administered.

A peptide of the present invention may consist of the amino acid sequence (I), or alternatively may comprise the amino acid sequence (I) as the core sequence and have various types of substance such as, without limitation, a peptide or an amino acid or an analogue thereof, a polyethylene glycol, a sugar, a polysaccharide, a nucleotide, or the like, attached at the N- and/or C-terminus of or at any amino acid residue in said amino acid sequence. Substances such as, without limitation, fluorescent labels, biotin, streptavidin, digoxigenin (DIG), magnetic beads, latex beads, and gold colloids, may be attached at the N/C terminus or at any amino acid residue through an amino acid(s) or peptide, or at a position inside of the amino acid sequence using an available functional group. For example, when an additional peptide is attached to a peptide of the present invention, such an additional peptide may consist of 1 to 50 amino acids, for example, 1 to 20, 1 to 15, or 1 to 9 amino acids (here, the number mentioned above of amino acids is illustrative and not for purposes of limitation). In addition, such an additional peptide may be, but is not limited to, one having functions as a histidine tag, a GST tag, an S tag, a Myc tag, an HA tag, or an E tag.

In addition to the peptides as described above, peptides of the present invention also include a peptide having an amino acid sequence in which one or more (for example, 2, 3, 4, or 5) amino acids are deleted, substituted, or added in the amino acid sequence of any of the above-described peptides; and a peptide having an amino acid sequence which has a homology of 50% or more, preferably 60% or more, further preferably 70% or more, more preferably 80% or more, further more preferably 90% or more (for example, 95%, 97%, 98%, or 99% or more), to the amino acid sequence of any of the above-described peptides. The homology between amino acid sequences can be determined, for example, using FASTA, BLAST, DNASIS (Hitachi Software Engineering Co., Ltd.), or GENETYX (Genetyx Corporation). Alternatively, in cases of short chain peptides, the homology can be calculated by simply comparing their amino acid sequences. Moreover, any of the amino acids in these sequences may be modified as appropriate. When having any of such amino acid sequences, the peptide of the present invention is one which is capable of binding to an immunoglobulin and has a higher water solubility than that of a peptide having the amino acid sequence (a) of SEQ ID No: 1.

The peptides of the present invention can be produced and obtained by various methods known to those skilled in the art. For example, a peptide of the present invention may be chemically synthesized, for example, by means of peptide solid-phase synthesis methods, or produced by genetic engineering, based on the nucleotide sequence encoding the peptide, or obtained in combination of these procedures.

The present invention, in an additional aspect thereof, is directed to a nucleic acid encoding a peptide of the present invention that is capable of binding to an immunoglobulin. In the specification, by nucleic acid is meant a single- or double-stranded DNA or RNA. Nucleic acids of the present invention can be produced and obtained by various methods known to those skilled in the art. The present invention, in an aspect thereof, is also directed to a nucleic acid having a nucleotide sequence encoding any of the amino acid sequences depicted in SEQ ID NOs: 9 to 15. In the present invention, as a nucleotide sequence encoding any of the amino acid sequences depicted in SEQ ID NOs: 9 to 15 are illustrated the nucleotide sequences depicted in SEQ ID NOs: 16 to 22. Therefore, the present invention is also directed to a nucleic acid having any of the nucleotide sequences depicted in SEQ ID NOs: 16 to 22. Also included in the present invention is a nucleic acid having a nucleotide sequence different from any of the above-described sequences, for example, a degenerate sequence, which encodes any of the amino acid sequences depicted in SEQ ID NOs: 9 to 15, but is not limited thereto.

Also, a nucleic acid of the present invention is, in addition to the nucleic acids described above, (a) a nucleic acid having a nucleotide sequence in which one or more (for example, 2, 3, 4, 5, 6, 7, 8, 9, 10, 11, 12, 13, 14, 15, 16, or 17) nucleotides are deleted, substituted, or added in the nucleotide sequence of any of the above-described nucleic acids; (b) a nucleic acid capable of formation of a hybrid with any of the above-described nucleic acids or a complementary strand thereof under stringent conditions; (c) a nucleic acid having a nucleotide sequence which has a homology of 50% or more (for example, 60%, 70%, 80%, 90%, 95%, 97%, 98%, 99% or more) to the nucleotide sequence of any of the above-described nucleic acids; or the like. Moreover, included as a nucleic acid of the present invention may be one in which any of the nucleotides in any of the above-described nucleic acids is be modified as appropriate. When having any of such nucleotide sequences, the nucleic acid of the present invention encodes a peptide that is capable of binding to an immunoglobulin and has a higher water solubility than that of a peptide having the amino acid sequence (a) of SEQ ID No: 1.

The above-mentioned stringent conditions include, for example, conditions as described in J. Sambrook et al., "Molecular Cloning: A Laboratory Manual, Second Edition", 1989, Cold Spring Harbor Laboratory Press, such as, for example, conditions in which after hybridization is done with a given probe at 68°C in a solution containing 6 × SSC, 5 × Denhardt's solution, 0.5% SDS, and 100 μg/ml denatured salmon sperm DNA, washing is carried out by changing washing conditions from those at room temperature in 2 × SSC, 0.1% SDS to those at 68°C in 0.1× SSC, 0.5% SDS; or washing at 65°C is carried out twice for 15 minutes each in a solution containing 2 × SSPE (which is described in Frederick M. Ausubel et al., "Current Protocols in Molecular Biology", 1987, John Wiley & Sons, Hoboken NJ.) and 0.1% SDS, subsequently twice for 15 minutes each in a solution containing 0.5 × SSPE and 0.1% SDS, and then twice for 15 minutes each in a solution containing 0.1 × SSPE and 0.1% SDS; or washing at 65°C is carried out twice for 15 minutes each in a solution containing 2 × SSPE, 0.1% SDS and formamide (5 to 50%), subsequently twice for 15 minutes each in a solution containing 0.5 × SSPE, 0.1% SDS and formamide (5 to 50%), and then twice for 15 minutes each in a solution containing 0.1 × SSPE, 0.1% SDS and formamide (5 to 50%).

The homology to an above-described nucleotide sequence can be determined, for example, using FASTA, BLAST, DNASIS (Hitachi Software Engineering Co., Ltd.), or GENETYX (Genetyx Corporation).

A nucleic acid of the present invention is any nucleic acid having a nucleotide sequence encoding a peptide that is capable of binding to an immunoglobulin. Also, a nucleic acid of the present invention may consist of an above-described nucleotide sequence, or alternatively may comprise an above-described nucleotide sequence as the core sequence and have various types of substance, such as, a nucleotide or a polynucleotide or an analogue thereof, attached at the 5'- and/or 3'-terminus of said nucleotide sequence. For example, when a polynucleotide is attached to a nucleic acid of the present invention, such a polynucleotide may consist of 1 to 150 nucleotides, for example, 1 to 60, 1 to 45, or 1 to 18 nucleotides. (Here, the number of nucleotides mentioned above is illustrative and not for purposes of limitation.)

The present invention, in an additional aspect thereof, is directed to a vector containing an above-described nucleic acid. The vector of the present invention may be any type of vector, if it contains an above-described nucleic acid. Vectors can be selected or designed as appropriate and obtained, depending on various factors, such as the type of hosts into which they are to be introduced, the required product, the purpose of their use, and others. Vectors of the present invention can also be used, for example, as a vector for the expression of a fusion protein in which a peptide capable of binding to an immunoglobulin is added at the N- or C-terminus of a given protein, by inserting a nucleotide sequence encoding the given protein, in frame at the 5' or 3' end of the nucleotide sequence depicted in any of SEQ ID NOs: 16 to 22. In order to facilitate the isolation of the given protein from the fusion protein, the vector of the present invention may contain a sequence recognized by a protease, such as HRV 3C, thrombin, Factor Xa, or enterokinase, between the peptide encoding nucleotide sequence as described above and the site at which the nucleotide sequence encoding the given protein is inserted. The vectors of the present invention may be introduced into cells to produce the peptide or fusion protein of the present invention.

The present invention, in another aspect thereof, is directed to a fusion protein in which a peptide of the present invention that is capable of binding to an immunoglobulin is added at the N-terminus and/or C-terminus of a given protein. Fusion proteins of the present invention can be produced and obtained by various methods known to those skilled in the art. A fusion protein of the present invention, which contains a peptide capable of binding to an immunoglobulin, allows the peptide to be used as a tag sequence, so as to isolate and/or purify the given protein, for example. In addition, a peptide of the present invention does not exhibit a high ability to bind to an immunoglobulin, comparable to that of Protein A or Protein G, and thus a fusion protein of the present invention, for example, when having been immobilized on a purification column, allows purification of the immunoglobulin under milder conditions, relative to those where Protein A or Protein G is used, and repeated use (that is, prevention of deterioration) of such a column, and has advantages, such as that reprobing is easy when used as a probe for detection of the immunoglobulin.

A given protein which is to be contained in a fusion protein of the present invention may be any kind of protein. When a fusion protein of the present invention contains, as a given protein, for example, an enzyme such as alkaline phosphatase (ALP), peroxidase (HRP), luciferase, a fluorescent protein, for example, green fluorescence protein (GFP) or the like, β-galactosidase, glutathione S-transferase, or maltose binding protein, such a fusion protein will facilitate, for example, the detection of binding between the peptide capable of binding to an immunoglobulin which is contained in the fusion protein and the immunoglobulin. A fusion protein of the present invention may also contain a tag sequence, such as a histidine tag, a GST tag, an S tag, a Myc tag, an HA tag, or an E tag, a nuclear localization signal, a silica binding protein, Protein A, or the like.

A fusion protein of the present invention may contain a peptide recognized by a protease, between the peptide of the present invention and the given protein. By containing such a peptide, the given protein can be isolated from the fusion protein without difficulty.

The present invention, in an additional aspect thereof, is directed to a nucleic acid encoding an above-described fusion protein.

Further, the present invention is directed to a vector containing a nucleic acid encoding an above-described fusion protein. Such a vector may be introduced into cells to produce a protein, whereby the fusion protein of the present invention can be obtained.

The present invention, in an aspect thereof, is directed to a cell containing a nucleic acid encoding an above-described peptide capable of binding to an immunoglobulin, a nucleic acid encoding an above-described fusion protein, or a vector containing one of these nucleic acids. Cells of the present invention can be generated by transforming host cells, such as, Escherichia coli, yeast, insect or animal cells, with the nucleic acid or vector as described above. A peptide or fusion protein of the present invention can also be produced by culturing a cell of the present invention and collecting the produced peptide or peptide-containing fusion protein, the peptide being capable of binding to an immunoglobulin.

The present invention, in an additional aspect thereof, is directed to a method for producing a peptide capable of binding to an immunoglobulin, the method comprising the steps of:

(a) transforming a cell with a vector which contains a nucleic acid encoding the peptide capable of binding to the immunoglobulin, and
(b) culturing the cell, thereby to produce the peptide. The transformation of cells can be carried out by means and/or methods known to those skilled in the art.

Therefore, the present invention is directed to a peptide that is capable of binding to an immunoglobulin and has improved water solubility, wherein the peptide is obtainable by the above-described method for peptide production.

The present invention, in another aspect thereof, is directed to a method for producing a fusion protein in which a peptide capable of binding to an immunoglobulin is added at the N- and/or C-terminus of a given protein, the method comprising the steps of:

(a) transforming a cell with a vector which contains a nucleic acid encoding the fusion protein in which the peptide capable of binding to the immunoglobulin is added at the N- and/or C-terminus of the given protein, and
(b) culturing the cell, thereby to produce the fusion peptide. The method for fusion-protein production of the present invention may further comprise the step of removing the given protein from the fusion protein.

Therefore, the present invention is directed to a fusion protein in which a peptide that is capable of binding to an immunoglobulin and has improved water solubility is added at the N-terminus and/or C-terminus of a given protein, wherein the fusion protein is obtainable by the above-described method for fusion-protein production.

The present invention, in an additional aspect thereof, is directed to a composition for allowing binding of an immunoglobulin, comprising a peptide capable of binding to an immunoglobulin or a fusion protein containing such a peptide. A component(s) other than the peptide or fusion protein may be selected as appropriate, depending on various factors, such as the purpose for which the composition is used. A composition of the present invention, which as mentioned above, comprises a peptide capable of binding to an immunoglobulin, may be, for example, a composition for determining the presence or the amount of the immunoglobulin, or a composition for isolating the immunoglobulin. Therefore, a composition of the present invention will make it possible, for example, to determine the amount of the immunoglobulin in a sample, to isolate the immunoglobulin from a sample.

The present invention, in another aspect thereof, is directed to a means for allowing binding of an immunoglobulin, wherein a peptide capable of binding to the immunoglobulin or a fusion protein containing such a peptide has been immobilized. A means of the present invention is one in which an above-described peptide or fusion protein of the present invention has been immobilized on a carrier, such as a plate, a resin, a column, a bead, a sugar-containing resin such as agarose or sepharose, a silica substrate, glass (slide glass and others), a metal (gold and others), or apatite. Immobilization can be effected using means and methods known to those skilled in the art, such as methods of immobilization via an amino or carboxyl group of the peptide or protein, via an SH group of an amino acid side chain, by ionic binding, and by hydrophobic binding.

Means of the present invention, which as mentioned above, are ones in which a peptide capable of binding to an immunoglobulin has been immobilized, include a means for determining the presence or the amount of the immunoglobulin and a means for isolating the immunoglobulin. A means of the present invention can also be used, for example, in ELISA plates, immunoglobulin purifying columns, immunoglobulin detecting glass arrays, microfluidic systems, SPR sensor chips, silica substrates for detection, systems for purification of antibody pharmaceuticals, and others.

The present invention, in an additional aspect thereof, is directed to a method for allowing binding of an immunoglobulin, which comprises the steps of:

(a) adding to a sample a peptide capable of binding to the immunoglobulin, or a fusion protein containing such a peptide; and
(b) determining a complex between the peptide or fusion protein and the immunoglobulin.

The sample may be any sample which is likely to contain the immunoglobulin. By determining the presence and/or the amount of a complex with the immunoglobulin, it is possible to determine whether the immunoglobulin is present in the sample, and furthermore, the amount of the immunoglobulin present in the sample. A peptide or fusion protein for use in the method of the present invention may be labeled. Labels include various ones known to those skilled in the art, such as biotinylation, and fluorescent, RI, and enzyme labels. Attachment of such a label facilitates examining a complex with the peptide or fusion protein. In addition, the method of the present invention may comprise the step of isolating the immunoglobulin from the complex.

Therefore, the present invention is directed to a kit for use in the method for allowing binding of an immunoglobulin, wherein the kit contains a peptide capable of binding to the immunoglobulin or a fusion protein comprising such a peptide. A kit of the present invention may comprise, for example, a label, a means to determine the complex, and others, in addition to the peptide or fusion protein.

The present invention, in another aspect thereof, is directed to a pharmaceutical composition for the treatment or prevention of a disease caused by binding between Clq and an immunoglobulin, comprising, as the active ingredient, a peptide of the present invention that is capable of binding to the immunoglobulin, a fusion protein containing such a peptide, a nucleic acid encoding such a peptide or fusion protein, or a vector containing such a nucleic acid. By a disease caused by binding between Clq and an immunoglobulin, is meant a disease resulted directly or indirectly from said binding, including, for example, immune-complex diseases, such as rheumatoid arthritis, arthritis, systemic lupus erythematosus (SLE), vasculitic syndrome, and nephritis, other inflammatory diseases, psoriasis, and malignant tumors. A pharmaceutical composition of the present invention allows the above-described diseases to be treated and prevented through the inhibition of the binding between C1q and the immunoglobulin by the peptide, contained in the composition, that is capable of binding to the immunoglobulin.

In this connection, since the peptide of the present invention is derived from C1q which is originally present within the human body, adverse effects do not or scarcely occur which arise from the administration of the peptide of the present invention to a human. Further, since the peptide of the present invention has improved water solubility by modification of the natural protein-derived amino acid sequence, the peptide has, for example, an advantage that the virtual dosage amount is increased when administered to a subject as a pharmaceutical composition. In addition, since a D-amino acid(s) is (are) comprised as a constituent, thereby enhancing the resistance to proteolytic degradation, the peptide of the present invention has an increased stability and a prolonged half-life within the body of a subject to whom the peptide is administered.

A peptide of the present invention which can be used as the active ingredient of a pharmaceutical composition is a peptide capable of binding to an immunoglobulin, wherein the peptide has an amino acid sequence represented by:

$$\text{Pro-Gly-Leu-}X_4\text{-}X_5\text{-Phe} \qquad \text{(I)}$$

wherein $X_4$ and $X_5$ are not Tyr at the same time. An example of a preferable peptide of the present invention for use as the active ingredient of a pharmaceutical composition is a peptide in which both $X_4$ and $X_5$ in the amino acid sequence (I) are hydrophilic amino acids. Such hydrophilic amino acids include Lys, Arg, His, Asp, Glu, Asn, Gln, Ser, Thr, and others. An example of a more preferable peptide of the present invention for use as the active ingredient of a pharmaceutical composition is a peptide having the amino acid sequence depicted in any of SEQ ID NOs. 9 to 15. Further preferable is a peptide having an amino acid sequence in which one or more amino acids except Gly, or all of the amino acid sequence except Gly, in the amino acid sequence represented by Formula (I), are substituted by the Corresponding D-amino acids(s).

A method by which a pharmaceutical composition of the present invention is administered may be selected as appropriate, depending on factors, such as the type of disease, the condition of the subject, and/or the target site. Such a method include, but is limited to, intradermal, subcutaneous, intramuscular, intravenous, transnasal, oral, and transpulmonary administration, suppository, and others. The amount of the peptide contained in a pharmaceutical composition of the present invention, dosage form of a pharmaceutical composition, frequency of administration, and the like may be selected as appropriate, depending on factors, such as the type of disease, the condition of the subject, and/or the target site. For example, the dosage amount of the peptide per administration may be, but is not limited to, 0.01 to 100 mg/kg, preferably 0.1 to 10 mg/kg.

The present invention, in an additional aspect thereof, is directed to a method for the treatment or prevention of a disease caused by binding between C1q and an immunoglobulin, the method comprising administering to a subject an effective amount of a peptide capable of binding to an immunoglobulin, of a fusion protein containing such a peptide, of a nucleic acid encoding such a peptide or fusion protein, or of a vector containing such a nucleic acid.

The present invention, in further another aspect thereof, is directed to a peptide capable of binding to an immunoglobulin, to a fusion protein containing such a peptide, to a nucleic acid encoding such a peptide or fusion protein, or to a vector containing such a nucleic acid for use in the treatment or prevention of a disease caused by binding between C1q and an immunoglobulin.

The present invention, in an additional aspect thereof, provides a use of a peptide capable of binding to an immunoglobulin, of a fusion protein containing such a peptide, of a nucleic acid encoding such a peptide or fusion protein, or of a vector containing such a nucleic acid for the manufacture of a medicament for use in the treatment or prevention of a disease caused by binding between C1q and an immunoglobulin.

The present invention will be described specifically and in detail below by way of Examples, which are not to be interpreted as limiting of the present invention.

Example 1

Evaluation of Solubility of Peptides in Water

Although the present inventors made investigation on peptides having C1q-derived amino acid sequences and found that a peptide having the amino acid sequence depicted in SEQ ID NO: 1, Pro-Gly-Leu-Tyr-Tyr-Phe, and modified versions thereof exhibit treatment and prevention effects in diseases caused by binding between C1q and immunoglobulins, for example, rheumatoid arthritis and immune complex diseases such as systemic lupus erythematosus (SLE), glomerulonephritis, vasculitis, and arthritis (see, International Publications WO2009/025300 and WO2010/084851), the present inventors made the investigation described below, in order to further increase the solubility of the peptide in water.

(1) Examination of Mutation Sites

With a peptide having the amino acid sequence depicted in SEQ ID NO: 1 that is capable of binding to an immunoglobulin and exhibits effects of treatment and prevention, sites which are not involved in the capability of binding to an immunoglobulin and were allowed to be mutated were searched for by alanine screening.

Peptides having the amino acid sequences indicated in Table 1 were synthesized by GL Biochem (Shanghai).

Table 1

| Synthetic peptide | Amino acid sequence | SEQ ID No: |
|---|---|---|
| R1 | PGLYYF | 1 |
| R2 | AGLYYF | 2 |
| R3 | PALYYF | 3 |
| R4 | PGAYYF | 4 |
| R5 | PGLAYF | 5 |
| R6 | PGLYAF | 6 |
| R7 | PGLYYA | 7 |

Human C1q protein (Carbiochem) was dissolved in 10 mM HEPES, 300 mM NaCl, 40% glycerol (pH 7.2) to prepare a 200 $\mu$g/ml solution of human C1q protein. The solution of human C1q protein was spotted at a volume of 2 $\mu$l (400 ng) per spot onto a nitrocellulose membrane of a size of 5 mm by 15 mm (Hybond C; Amersham), which was then air-dried at room temperature for about one hour. The nitrocellulose membrane was soaked in TBS, incubated for 5 minutes, and blocked at room temperature for one hour using TBS (20 mM Tris-HCl pH 7.5, 150 mM NaCl) containing 5% of a blocking agent (Amersham ECL blocking reagent; GE Healthcare). After washing lightly in TBS, the membrane was soaked in 20 $\mu$l of a solution of alkaline phosphatase (ALP)-labeled human immunoglobulin (IgG) (BECKMAN COULTER) which was diluted 1,000 times with TBS and mixed with each peptide solution to make a peptide concentration of 500 $\mu$g/ml, and subjected to reaction at room temperature for one hour. After washing lightly in a TTBS solution (TBS to which Tween 20 was added to reach a final concentration of 0.05%), the membrane was washed in the same solution three times for 10 minutes each, with shaking.

ALP-labeled immunoglobulin (IgG) was used to detect a complex between human C1q protein and IgG. After the nitrocellulose membrane was washed lightly in an ALP color development buffer (Tris-HCl pH 9.5 containing 100 mM NaCl, 5 mM MgCl$_2$), the membrane was soaked in 30 $\mu$l of the ALP color development buffer containing a BCIP/NBT solution (Promega), and subjected to color development at room temperature for 10 minutes. When color developments were achieved, the nitrocellulose membrane was soaked in a sufficient volume of distilled water to wash away the color development buffer. After washing, the nitrocellulose membrane was air-dried and the color developments were captured using a Multi Gauge (FUJIFILM).

The results are shown in Fig. 1. In peptides in which the 3rd to 5th amino acids in the amino acid sequence depicted in SEQ ID NO: 1 were substituted one-by-one by alanine, these peptides were found to retain immunoglobulin binding ability. It was demonstrated that the immunoglobulin binding ability was almost not affected, particularly when the 4th or 5th amino acid was substituted by an amino acid which is of a kind different from that of each of the original amino acids.

(2) Examination of Introducing Hydrophilic Amino Acid into Peptide

Water solubility was evaluated for peptides in which various kinds of hydrophilic amino acid were substituted for the 4th and 5th amino acids which were verified in (1) to be allowed to be mutated. The peptides listed in Table 2 were synthesized by GL Biochem (Shanghai). Among the peptides listed in Table 2, peptides SP1 to SP7 are peptides in which various kinds of hydrophilic amino acid were substituted for the 4th and 5th amino acids.

Table 2

| Synthetic peptide | Amino acid sequence | SEQ ID No: |
|---|---|---|
| R1 | PGLYYF | 1 |
| SP1 | PGLDKF | 9 |
| SP2 | PGLDRF | 10 |
| SP3 | PGLDHF | 11 |
| SP4 | PGLEKF | 12 |

(continued)

| Synthetic peptide | Amino acid sequence | SEQ ID No: |
|---|---|---|
| SP5 | PGLERF | 13 |
| SP6 | PGLEHF | 14 |
| SP7 | PGLSSF | 15 |

The results are shown in Table 3. In Table 3, the circle symbol is assigned to a peptide which was dissolved in a solvent (PBS or physiological saline) at a concentration of 10 mg/ml or higher, and the cross symbol is assigned to a peptide which was not dissolved at a concentration of 1 mg/ml. The peptide having the original unmodified sequence in the amino acid sequence depicted in SEQ ID NO: 1 (R1) did not dissolve in these solvents at a concentration of 1 mg/ml, whereas the peptides which had hydrophilic amino acids substituted for the 4th and 5th amino acids from the N-terminus dissolved sufficiently even at a concentration of 10 mg/ml or higher. In the subsequent experiments, the peptides in which the 4th and 5th amino acids were substituted by hydrophilic amino acids were used.

In general, in cases of peptide pharmaceuticals, methods of administration by intravenous or subcutaneous injection are often employed in view of their easy degradation in the digestive tract. For this reason, peptides having low water solubility have difficulties in their clinical application. Then, oral administration, even when selected, will cause a problem that these peptides are prone to degradation. In contrast, the peptides of the present invention, which have increased water solubility, allow intravenous and subcutaneous injection at high doses. In addition, peptides of the present invention, which have increased water solubility, have an advantage of being easily used as materials.

Table 3

| Synthetic peptide | Amino acid sequence | Result |
|---|---|---|
| R1 | PGLYYF | × |
| SP1 | PGLDKF | ○ |
| SP2 | PGLDRF | ○ |
| SP3 | PGLDHF | ○ |
| SP4 | PGLEKF | ○ |
| SP5 | PGLERF | ○ |
| SP6 | PGLEHF | ○ |
| SP7 | PGLSSF | ○ |

Example 2

Verification of Immunoglobulin Binding Ability of Peptides having Water Solubility imparted thereto
The capability of binding to an immunoglobulin was verified for the peptide obtained in Example 1-(2).

Each of peptides SP1 to SP7 was dissolved in DMSO to make a concentration of 10 mg/ml. In addition, solutions were prepared in a similar way and used in the experiments described below, also in the case of peptides having the following amino acid sequences: STGKFTCKVPGLYYF (peptide R8), and dPGdLdYdYdF and dSdKdVdPGdLdYdYdF (wherein "dL", "dP", "dA", "dY", "dF", "dS", "dK", "dV", and "dT" denote the D-form of leucine, proline, alanine, tyrosine, phenylalanine, serine, lysine, valine, and threonine, respectively) (peptides R9 and R10, respectively), for which the capability of binding to an immunoglobulin has been verified in PCT International Publications WO 2009/025300 and WO 2010/084851.

Human C1q protein (Carbiochem) was dissolved in 10 mM HEPES, 300 mM NaCl, 40% glycerol (pH 7.2) to prepare a 200 $\mu$g/ml solution of human C1q protein. The solution of human C1q protein was spotted at a volume of 2 $\mu$l (400 ng) per spot onto a nitrocellulose membrane of a size of 5 mm by 15 mm (Hybond C; Amersham), which was then air-dried at room temperature for about one hour. The nitrocellulose membrane was soaked in TBS, incubated for 5 minutes, and blocked at room temperature for one hour using TBS (20 mM Tris-HCl pH 7.5, 150 mM NaCl) containing 5% of a blocking agent (Amersham ECL blocking reagent; GE Healthcare). After washing lightly in TBS, the membrane was soaked in 20 $\mu$l of a solution of alkaline phosphatase (ALP)-labeled human immunoglobulin (IgG) (BECKMAN COULTER) which was diluted 1,000 times with TBS and mixed with each peptide solution to make a peptide concentration

of 500 μg/ml, and subjected to reaction at room temperature for one hour. After washing lightly in a TTBS solution (TBS to which Tween 20 was added to reach a final concentration of 0.05%), the membrane was washed in the same solution three times for 10 minutes each, with shaking.

ALP-labeled immunoglobulin (IgG) was used to detect a complex between human C1q protein and IgG. After the nitrocellulose membrane was washed lightly in an ALP color development buffer (Tris-HCl pH9.5 containing 100 mM NaCl. 5 mM $MgCl_2$), the membrane was soaked in 30 μl of the ALP color development buffer containing a BCIP/NBT solution (Promega), and subjected to color development at room temperature for 10 minutes. When color developments were achieved, the nitrocellulose membrane was soaked in a sufficient volume of distilled water to wash away the color development buffer. After washing, the nitrocellulose membrane was air-dried and the color developments were captured using a Multi Gauge (FUJIFILM) for quantification.

The results are shown in Fig. 2. In the figure, NC represents the result from samples in which peptide-free DMSO was added to reaction solutions which did not contain human immunoglobulin (IgG) labeled with alkaline phosphatase (ALP), and PC represents the result from samples in which peptide-free DMSO was added to reaction solutions which contained human immunoglobulin (IgG) labeled with alkaline phosphatase (ALP). Each of the peptides in which hydrophilic amino acids were substituted for the 4th and 5th amino acids in the amino acid sequence depicted in SEQ ID NO: 1 inhibited the binding between C1q and the immunoglobulin.

Example 3

Suppressing effects of peptides of the present invention on the production of inflammation associated proteins induced by TNF-α stimulation Using real-time RT-PCR method, an investigation was made as to whether the addition of peptides of the present invention resulted in the decrease of inflammation associated proteins such as cytokines and extracellular proteases which were produced by TNF-α stimulation of synovial cells collected from a human RA patient.

DMEM medium (GIBCO) containing 10% FBS, 1% L-Glu, and 1% P/S was used to culture synovial cells (passages 3 to 5) in 10-cm diameter culture dishes until 80% to 90% confluence was reached. Cultured cells were seeded into 24-well plates at a density of $5 \times 10^5$ cells/well and incubated for 24 hours. A 10-times dilution of 10 μg/ml rhTNF-α (R&D Recombinant Human TNF-α/TNFSF1A, #210-TA) was added at 10 μl/well to stimulate the synovial cells. Solutions of the respective peptides of the present invention were prepared to predetermined concentrations using physiological saline (Otsuka Pharmaceutical Co., Ltd.) and added at 10 μl/well, so that the amount of each of the peptides added was 1, 10, or 100 μg, and incubation was carried out for 24 hours. The medium was removed, and the cells were washed with PBS(-). TRIzol (invitrogen TRIzol Reagent, #15596-018) was added at 1 ml/well, and the cells were scraped off with a cell scraper and collected into 1.5-ml Eppendorf tubes. After RNA extraction, cDNA was prepared using High-Capacity cDNA Reverse Transcription Kits (Applide Biosystems) and the amounts of expression of genes were semi-quantitatively determined for their assessment by real-time RT-PCR (ABI PRISM 7900HT).

The results are shown in Fig. 3. The peptides of the present invention, such as SP3 and SP4, were demonstrated to suppress the production of inflammation associated proteins, such as IL-1β, TNF-α, MMP-1, and MMP-3, which were induced by TNF-α stimulation.

Example 4

Arthritis Suppressing Effects of Peptides of The Present Invention in Mice Monoclonal Antibody Cocktail-induced Arthritis Model

1. Materials and Methods

1-1 Mice (BALB/c Cr Slc (SPF)) were used to examine arthritis suppressing effects of peptides of the present invention on monoclonal antibody cocktail-induced arthritis. Mice were fed normal chow and allowed to acclimate for 6 days before used in experiments.

1-2 Amino acid sequences of peptides used in experiments

- peptide R1: PGLYYF (test substance a)
- peptide R9: dPGdLdYdYdF (test substances α)
- peptide SP4: PGLEKF (test substance VII-1)
- a variant of peptide SP4 composed of the corresponding D-amino acids (except for glycine): dPGdLdEdKdF (test substance VII-2)

1-3 Outline of Experiments

Arthritis was caused by intravenous administration of 2 mg/animal of an arthritis-inducing monoclonal antibody cocktail and three days later, intraperitoneal administration of 50 μg/animal of lipopolysaccharide (LPS). Peptides R1 and R9 each were intraperitoneally administered at a dose of 10 mg/kg once a day from the day following the LPS administration (day 4) to day 14. Peptide SP4 and the variant of peptide SP4 composed of the corresponding D-amino acids (except for glycine) each were intraperitoneally administered at a dose of 10 or 100 mg/kg once a day from the day following the LPS administration (day 4) to day 14. A positive control agent, methotrexate (MTX), was intraperitoneally administered at 0.15 mg/kg once a day from day 4. A control group was given physiological saline at 10 mL/kg once a day from day 4. On even days from day 0 to day 14, the extremities of each of the animals were observed for clinical score and the hind-limb volume was determined.

In addition, preparation of joint tissue specimens was carried out and soft X-ray radiographs were taken in the following way. Under anesthesia, blood was removed using heparin-containing physiological saline, and an ice-cold, 4% solution of paraformaldehyde was continuously injected to perfusion-fix the whole body. After perfusion-fixation, the knee joints (obtained by cutting at the center of the femur and of the tibia and removing the skin and muscles) and the hock joints (from the center of the tibia to the toe tips) of both hind limbs were excised and further fixed by immersion in a 4% solution of paraformaldehyde overnight (4°C). After that, both of the knee joints and both of the hock joints were transferred into 50 mM PBS (4°C), and then subjected to soft x-ray radiography of the hock joints in two directions (from the internal side and from the upper side).

1-4 Preparation of Peptide Solutions

A solution of each of peptides R1 and R9 was prepared by weighing the peptide into an agate mortar, grinding it with a pestle, and then gradually adding physiological saline to form a suspension and make a concentration of 1 mg/ml. The preparations were cooled and stored until use. For peptide SP4 and the variant of peptide SP4 composed of the corresponding D-amino acid counterparts (except for glycine), solutions were prepared by adding 1.0 ml of physiological saline to dissolve each of these peptides, so as to make concentrations of 10 and 1 mg/ml. The preparations were cooled and stored until use.

1-5 Preparation of Methotrexate Solution

Methotrexate (1 mg) was weighed and dissolved by adding 1 ml of a 0.1 M sodium hydroxide solution. To 0.15 ml of the 1-mg/ml solution prepared, was added 9.85 ml of physiological saline, to prepare a solution of a concentration of 0.015 mg/ml. The resulting solution was cooled and stored until use.

1-6 Body weight measurement, general observation of symptoms, and grouping

The body weights of the respective animals were measured on days 0, 3, 6, 9, 12, and 14 during the period of the examination, with day 0 being the day when the arthritis-inducing monoclonal antibody cocktail was administered. General observation of conditions was made on a daily basis.

1-7 Generation of an arthritis model

To forty 7-week-old mice, the arthritis-inducing monoclonal antibody cocktail was intravenously administered at a dose of 2 mg/animal on day 0, and LPS was intraperitoneally administered at a dose of 50 μg/animal on day 3.

1-8 Administration of Peptide Solutions

Each of the peptide solutions was given via intraperitoneal administration at 10 or 100 mg/kg once a day from day 4. As a control, physiological saline was intraperitoneally administered once a day from day 4, and the volume of administration was 10 ml/kg. Methotrexate was intraperitoneally administered at 0.15 mg/kg once a day from day 4, and the volume of administration was 10 ml/kg. Needles (26G; Terumo) and syringes (1.0-ml capacity; Terumo) were used for administration.

1-9 Observation of Clinical Score

Observation of clinical score was performed on even days from day 0 to day 14 as follows. The total score for the extremities was set to a maximum of 12. Visual observation of swelling of the legs was performed.

<Clinical Scores>

0: normal joint
1: slight inflammation and redness
2: serious erythema and swelling over the whole limb and preventing the use of the limb
3: deformation of the limb or joint accompanied by ankylosis, joint stiffness, loss of function

1-10 Examination Groups

Examination groups were as indicated below:

Table 4

| Group | Dose | Administration route | Sex and number of animals used |
|---|---|---|---|
| Control | - | Intraperitoneal | |
| Test substance a | 10 mg/kg | Intraperitoneal | |
| Test substance $\alpha$ | 10 mg/kg | Intraperitoneal | |
| Test substance VII-1 | 10 mg/kg | Intraperitoneal | |
| Test substance VII-1 | 100 mg/kg | Intraperitoneal | 5 male animals per group |
| Test substance VII-2 | 10 mg/kg | Intraperitoneal | |
| Test substance VII-2 | 100 mg/kg | Intraperitoneal | |
| Methotrexate | 0.15 mg/kg | Intraperitoneal | |

1-11 Methods for Statistical Analysis

Results of examinations are expressed by mean value $\pm$ standard error and were analyzed using EXSAS (Version 7.1.6, Arm Systex Co., Ltd.). Clinical scores were subjected to Wilcoxon's test. Quantitative data, such as body weights and leg volumes, were subjected to Bartlett's test, followed by Dunnett's multiple comparison test if the variance was uniform or by Steel's multiple comparison test if the variance was not uniform.
The level of significance was set at 5% on both sides.

2. Results

2-1 Body Weight Changes and General Conditions of Animals

The body weight in the control group was 22.5 $\pm$ 0.2 g on day 0, and decreased to 18.1 $\pm$ 0.2 g on day 6, a day after the LPS administration. However, the body weight was gradually returned to 19.6 $\pm$ 0.3 g on day 9, to 21.1 $\pm$ 0.4 g on day 12, and to 22.2 $\pm$ 0.4 g on day 14. The body weights in the other groups receiving the test substances also showed changes similar to that seen in the control group, and no significant differences were observed between these groups. In addition, there were not observed any symptoms which seemed to be due to adverse effects for all the animals throughout the period of the examination.

2-2 Clinical Scores

The results are shown in Figs. 4 to 7.
The clinical score in the control group was 0.0 $\pm$ 0.0 on day 0 and 0.6 $\pm$ 0.2 on day 2, and increased over time to 3.0 $\pm$ 0.3 on day 4, a day after the LPS administration, to 6.6 $\pm$ 0.7 on day 6, to 6.8 $\pm$ 0.6 on day 8, to 8.4 $\pm$ 0.5 on day 10, to 11.0 $\pm$ 0.3 on day 12, and to 11.2 $\pm$ 0.4 on day 14. For the score on day 14, the score for each of the fore and hind-limbs was 2 or 3 in all the five animals, whereby a total score of 10 to 12 was obtained (Figs. 4 to 7).
The clinical score in the group receiving 10 mg/kg of test substance a showed changes at lower values during the period, with the highest value of 9.6 $\pm$ 0.8 on day 14, relative to those in the control group, while two animals were found to have a score of zero, whereby a total score of 7 to 12 was obtained (Fig. 4).

The clinical score in the group receiving 10 mg/kg of test substance α showed 7.0 ± 0.7 on day 12 and 7.4 ± 0.9 on day 14, which were significantly lower values, relative to those in the control group. On day 14, three animals were found to have a score of zero, whereby a total score of 5 to 10 was obtained (Fig. 5).

The clinical score in the group receiving 10 mg/kg of test substance VII-1 showed 7.2 ± 1.0 on day 12 and 7.4 ± 1.1 on day 14, which were significantly lower values, relative to those in the control group. On day 14, three animals were found to have a score of zero, whereby a total score of 5 to 10 was obtained (Fig. 6).

The clinical score in the group receiving 100 mg/kg of test substance VII-1 showed 7.2 ± 0.9 on day 12 and 8.0 ± 0.9 on day 14, which were significantly lower values, relative to those in the control group. On day 14, two animals were found to have a score of zero, whereby a total score of 5 to 10 was obtained (Fig. 6).

The clinical score in the group receiving 10 mg/kg of test substance VII-2 showed 5.0 ± 0.8 on day 10, 5.8 ± 0.9 on day 12, and 6.0 ± 1.0 on day 14, which were significantly lower values, relative to those in the control group. On day 14, four animals were found to have a score of zero, whereby a total score of 4 to 10 was obtained (Fig. 7).

The clinical score in the group receiving 100 mg/kg of test substance VII-2 showed 5.8 ± 0.5 on day 10, 6.8 ± 0.7 on day 12, and 7.2 ± 0.8 on day 14, which were significantly lower values, relative to those in the control group. On day 14, four animals were found to have a score of zero, whereby a total score of 4 to 8 was obtained (Fig. 7).

The clinical score in the group receiving 0.15 mg/kg of MTX showed 8.2 ± 0.9 on day 12 and 8.6 ± 0.9 on day 14, which were significantly lower values, relative to those in the control group. On day 14, one animal was found to have a score of zero, whereby a total score of 6 to 11 was obtained (Figs. 4 to 7).

2-3 Hind-Limb Volumes

The results are shown in Figs. 8 to 11 and Figs. 12 to 15.
The hind-limb volume in the control group was 0.212 cm$^3$ on day 0, and showed a greatly increased value on day 6, rather than on day 4, each being a day after the LPS administration. The hind-limb volume was 0.287 cm$^3$ on day 4 and 0.419 cm$^3$ on day 6, and reached the highest value of 0.460 cm$^3$ on day 8, and then decreased down to 0.331 cm$^3$ on day 14 (Figs. 8 to 11). When the volume on day 0 was set to be 100%, the increasing rate was 136.0% on day 4 and 198.3% on day 6, and reached the highest percentage of 217.8% on day 8, and decreased down to 156.8% on day 14 (Figs. 12 to 15).

The hind-limb volume in the group receiving 10 mg/kg of test substance a was 0.213 cm$^3$ on day 0 and 0.403 cm$^3$ (an increasing rate of 190.2%) on day 6, and reached the highest value of 0.452 cm$^3$ (the highest increasing rate of 213.2%) on day 8, and then gradually decreased down to 0.343 cm$^3$ (an increasing rate of 161.8%) on day 14: no significant difference was found in comparison to the control group (Fig. 8 and Fig. 12).

The hind-limb volume in the receiving 10 mg/kg of test substance α was 0.212 cm$^3$ on day 0 and 0.367 cm$^3$ (an increasing rate of 173.7%) on day 6, and reached the highest value of 0.455 cm$^3$ (the highest increasing rate of 215.1%) on day 8, and then gradually decreased down to 0.328 cm$^3$ (an increasing rate of 155.1%) on day 14. A significant decrease in the.volume on day 6 was found in comparison to the control group (Fig. 9 and Fig. 13).

The hind-limb volume in the receiving 10 mg/kg of test substance VII-1 was 0.223 cm$^3$ on day 0 and 0.368 cm$^3$ (an increasing rate of 165.2%) on day 6, and reached the highest value of 0.438 cm$^3$ (the highest increasing rate of 196.6%) on day 8, and then gradually decreased down to 0.332 cm$^3$ (an increasing rate of 149.1%) on day 14. Significant decreases in the volume and increasing rate on day 6 were found in comparison to the control group (Fig. 10 and Fig. 14).

The hind-limb volume in the receiving 100 mg/kg of test substance VII-1 was 0.219 cm$^3$ on day 0 and 0.362 cm$^3$ (an increasing rate of 165.5%) on day 6, and reached the highest value of 0.418 cm$^3$ (the highest increasing rate of 191.4%) on day 8, and then gradually decreased down to 0.329 cm$^3$ (an increasing rate of 150.3%) on day 14. Significant decreases in the volume and increasing rate on day 6 were found in comparison to the control group (Fig. 10 and Fig. 14).

The hind-limb volume in the receiving 10 mg/kg of test substance VII-2 was 0.211 cm$^3$ on day 0 and 0.363 cm$^3$ (an increasing rate of 172.0%) on day 6, and reached the highest value of 0.429 cm$^3$ (the highest increasing rate of 204.0%) on day 8, and then gradually decreased down to 0.340 cm$^3$ (an increasing rate of 161.8%) on day 14. A significant decrease in the volume and a decreasing trend of the increasing rate (P = 0.0753) on day 6 were found in comparison to the control group (Fig. 11 and Fig. 15).

The hind-limb volume in the receiving 100 mg/kg of test substance VII-2 was 0.223 cm$^3$ on day 0 and 0.373 cm$^3$ (an increasing rate of 167.4%) on day 6, and reached the highest value of 0.420 cm$^3$ (the highest increasing rate of 188.5%) on day 8, and then gradually decreased down to 0.356 cm$^3$ (an increasing rate of 159.6%) on day 14. A decreasing trend of the volume (P = 0.0876) and a significant decrease in the increasing rate on day 6 were found in comparison to the control group (Fig. 11 and Fig. 15).

The hind-limb volume in the receiving 0.15 mg/kg of MTX was 0.216 cm$^3$ on day 0 and 0.381 cm$^3$ (an increasing rate of 176.2%) on day 6, and reached the highest value of 0.441 cm$^3$ (the highest increasing rate of 204.2%) on day 8, and then gradually decreased down to 0.356 cm$^3$ (an increasing rate of 165.4%) on day 14. No significant difference was found in comparison to the control group (Figs. 8 to 11 and Figs. 12 to 15).

3. Discussion

The hind-limb volume in the control group began to increase on day 2, reached the highest value on day 8 (5 days after the LPS administration), and then gradually decreased. The hind-limb volume in the control group was found to greatly increase from day 4 to day 6. In comparison to the control group, significant decreases were found in the hind-limb volume and increasing rate on day 6 for the groups receiving 10 mg/kg of test substance $\alpha$, 10 and 100 mg/kg of test substance VII-1, and 10 and 100 mg/kg of test substance VII-2. These results indicate that these test substances exerted suppressive effects when edema became worst. On day 8 when the volume arrived at its highest value, the difference in the volume ratio relative to the control group was -13.6% for the group receiving MTX, a positive control group; -21.1% and -26.4% for the groups receiving 10 and 100 mg/kg of test substance VII-1, respectively; and -13.8% and -29.3% for the groups receiving 10 and 100 mg/kg of test substance VII-2, respectively: no significant difference was found in comparison to the control group, but suppressive effects on edema were found.

The clinical score in the control group began to get worse on day 2, and was found to have a biphasic score increase with an increase in the score associated with edema from day 4 to day 6, followed by joint stiffening from day 8 to day 14. In the first phase from day 4 to day 6, although a significant difference in the score was not found, the score was found to decrease in the groups receiving 10 mg/kg of test substance $\alpha$, and 10 and 100 mg/kg of test substance VII-2, in comparison to the control group. In the second phase from day 8 to day 14, a significant effect of improving the score was found on days 12 and 14 in the groups receiving 10 mg/kg of test substance $\alpha$, 10 and 100 mg/kg of test substance VII-1, and in the MTX administered group, a positive control group. In the groups receiving 10 and 100 mg/kg of test substance VII-2, a significant effect of improving the score was found on days 10, 12, and 14. The score on day 14 in the control group comprised scores of 2 or 3 for the fore- and hind-limbs in all the five animals, whereby a total score of 10 to 12 was obtained. In contract, in the groups receiving 10 and 100 mg/kg of test substance VII-2, the number of animals which were found to have a score of zero was 4 in each of these groups, whereby total scores of 4 to 10 and of 4 to 8 were obtained, respectively.

From the results described above, the arthritis suppressing effect of the test substances in this animal model was in the following order: VII-2 $\geq$ VI-II $\geq$ $\alpha$ > a. The arthritis suppressing effects of the test substances were equal to or greater than that of methotrexate: in particular, VII-2 and VII-1 exhibited a greater effect of suppressing arthritis. The reason for this would be because increased water solubility of test substances VII-2 and VII-1 is involved.

These results revealed that the peptides of the present invention have an arthritis suppressing effect superior to that of methotrexate, a pharmaceutical which is already in clinical use, and will be useful in the treatment and prevention of arthritis and related diseases. Note that methotrexate is known to cause serious adverse effects even when used in very small amounts. The peptides of the present invention, on the other hand, are derived from the natural substance and did not display any detectable adverse effects in the above-described examinations. Therefore, it can be said that the peptides of the present invention are more beneficial, relative to conventionally used pharmaceuticals, also in the viewpoint of safety.

Example 5

Arthritis Suppressing Effects of Peptides of The Present Invention in Adjuvant Arthritis Rat Model

Since the peptides of the present invention exhibited a good inflammation-suppressing effect in the monoclonal antibody cocktail arthritis mice model, the present inventors also examined the inflammation suppressing effect of a peptide of the present invention in an adjuvant arthritis rat model, which is a model for evaluation of systemic inflammation and is said to be a model which most closely simulates the pathological conditions of human rheumatoid arthritis.

1. Materials and Methods

1-1 Rats

Male Lewis/CrlCrlj (SPF) rats were used to investigate the arthritis suppressing effect of a peptide of the present invention on adjuvant-induced arthritis. Forty-five rats were purchased from Charles River Laboratories Japan, Inc., and fed normal chow and allowed to acclimate for 7 days before used (the range of body weights was from 193.3 to 218.3 g at the beginning of use).

1-2 Test substance

The peptide which was used in experiments was:

a variant of peptide SP4 composed of the corresponding D-amino acids (except for glycine): dPGdLdEdKdF (test substance VII-2)

1-3 Generation of Adjuvant Arthritis Model

In an agate motor, dead tubercle bacilli (Mycobacterium butyricum; Lot No. 0260570, Difco) were pulverized. Then, liquid paraffin was added in small amounts to spread the pulverized bacilli well, so as to allow them to be emulsified at 1.0 mg/mL. The resultant emulsion was used after verification to ensure that the pulverized bacilli had been allowed to be finely emulsified, using an NK MICRONIZER (Nippon Seiki Co., Ltd.).
A right hind-limb of the respective rats was exposed, and 100 μL of the emulsion was intradermally injected once into a footpad of the right hind-limb using a 25G-needle attached syringe, to cause arthritis (day 0). To a negative control group, liquid paraffin was injected in a similar way.

1-4 Examination Groups and Administration Methods

Preparation of the test substances and the examination groups were as indicated in Table 5. Each of the groups was comprised of 5 animals.

Table 5

| Group | Drug | Administration route | Dose (mg/kg) | Concentration of administration solution (mg/ml) |
|---|---|---|---|---|
| 1 | Negative control group | Oral (distilled water) | - | - |
| 2 | Arthritis (positive) control group | Intraperitoneal (physiological saline) | 0 | 0 |
| 3 | Methotrexate pre-administered group | Oral (distilled water) | 0.15 | 0.03 |
| 4 | Methotrexate group | Oral (distilled water) | 0.15 | 0.03 |
| 5 | Reflumide group | Oral (distilled water) | 10 | 2.0 |
| 6 | Test substance VII-2 low-dose group | Intraperitoneal (physiological saline) | 10 | 2.0 |
| 7 | Test substance VII-2 high-dose group | Intraperitoneal (physiological saline) | 100 | 20 |

Five animals were included in each group, and the volume of solutions administered was 5 ml/kg in all administrations.
Except for the methotrexate pre-administered group, each of the test substances was administered daily from 10 days after the adjuvant administration (day 10 post-adjuvant) to 21 days after the adjuvant administration (day 21 post-adjuvant) (a total of 11 administrations). To the methotrexate pre-administered group, methotrexate was administered prophylatically once before the adjuvant administration and daily on and after the day of the Adjuvant administration (a total of 21 administrations).
The day of examination was indicated on the basis in which the initial date was set to be the day when the adjuvant was injected (day 0 post-adjuvant).

1-5 Observation and Measurement Methods

(i) Measurement of Body Weights

Body weights for all living animals were measured in the morning on days 0, 3, 7, 10, 14, 17, and 21.

(ii) Measurement of Arthritis Scores

Arthritis was evaluated only for the hind-limbs, based on the criteria described below:

Regarding swelling in knuckle joints:

Score 0: no swelling
Score 1: swelling of not more than two digits
Score 2: swelling of three digits
Score 3: swelling of four digits
Score 4: swelling of all the digits

Regarding swelling in hind-limb insteps are:

Score 0: no swelling
Score 1: mild swelling
Score 2: modest swelling
Score 3: rather severe swelling
Score 4: severe swelling

Regarding the degree of redness:

Score 0: no redness
Score 1: mild redness
Score 2: modest redness
Score 3: rather severe redness
Score 4: severe redness

(iii) Measurement of Foot Volumes of Left And Right Hind-Limbs

Measurement was performed using a plethysmometer (UNICOM TK105). The increasing rate of foot volume (percentage of paw swelling, paw swelling %) was calculated by the following formula: Percent paw swelling (%) = [(Vt - Vo)/Vo] $\times$ 100 where
Vt: foot volume after adjuvant injection (mL)
Vo: foot volume before adjuvant injection (mL) (measured on day 0).
In addition, in these examinations, the percent change of edema relative to day 10 post-adjuvant was calculated, in order to assess the effect of treatment with the test substances.

(iv) Measurement of TNF-$\alpha$ and IL-1$\beta$ in Plasma

TNF-$\alpha$ and IL-1$\beta$ in plasma were measured in a double-point method using the TNF-$\alpha\beta$ ELISA kit (Lbis® TNF-$\alpha$-rat) and the IL-1$\beta$ ELISA kit (Quantikine® Rat IL-1$\beta$/IL-1F2).

1-6 Statistical Analysis

Statistical Analysis

Data are expressed by mean $\pm$ standard error. Since the uniformity of variance was not verified in testing between the negative and the positive control groups, testing for significant difference was performed by Aspin-Welch t-test. Since the uniformity of variance was not verified in testing for significant differences of the respective test-substance administered groups relative to the positive control group, assessment was performed by Dunnett's multiple comparison test after Kruskal-Wallis testing, using non-parametric procedures. Differences were considered to be statistically significant at p values of less than 0.05.

2. Results

2-1 Body Weights and Conditions of Animals

All the adjuvant administered groups showed a slight, but not statistically significant, suppression of the increase in body weight, in comparison to the negative control group. During the period of the examination, there were no animals displaying deaths, abnormalities or symptoms which seemed to be due to adverse effects.

2-2 Increasing rate in Foot Volume (Tables 6a, 6b, 7a, and 7b)

The negative control group was found to show normal increases in foot volume with growth in both the left and right hind-limbs and the increasing rate in foot volume on day 21 post-adjuvant was 9.20% for the left hind-limb and 7.65% for the right hind-limb. The arthritis control group was found to show remarkable increases over time in foot volume caused by the adjuvant in both the left and right hind-limbs, and had significant increases in foot volume in both the hind-limbs on and after day 3, in comparison to the control group, and the increasing rate in foot volume on day 21 post-adjuvant was 22.03% for the left hind-limb and 53.04% for the right hind-limb.

The methotrexate pre-administered group showed changes in the left hind-limb similar to those seen in the negative control group, until day 14 post-adjuvant. Although a significant effect of suppressing the increase in foot volume was found only on day 14 post-adjuvant, the foot volume rapidly increased on day 17 and day 21 post-adjuvant, and the increasing rate in foot volume on day 21 post-adjuvant was 12.47% and had a greater difference from that in the negative control group. The increasing rate in the right hind-limb showed an increase over time and was at the highest 70% or less of that in the arthritis control group, and was found to have a significant effect of suppressing the increase in foot volume on day 14 post-adjuvant.

In the methotrexate administered group in which methotrexate administration was started on day 10 post-adjuvant, the increasing rate in foot volume in the left hind-limb, relative to day 10 post-adjuvant, did not change at all on day 14 post-adjuvant, but turned to increase on day 17 post-adjuvant and were comparable to those in the arthritis control group on day 21 post-adjuvant. During the period of from day 10 to day 21 post-adjuvant, the right hind-limb showed a significant decrease on day 14 post-adjuvant, and the increasing rate in foot volume on the other days, relative to day 10 post-adjuvant, did not show large changes.

In the leflumide group, the increasing rate in foot volume in the left and right hind-limbs, relative to day 10 post-adjuvant, were not found to show large changes and changed to negative values for both the hind-limbs on day 21 post-adjuvant, relative to day 10 post-adjuvant.

In the group receiving 10 mg/kg of test substance VII-2, a remarkable and significant suppression of the increase in foot volume was found in both the hind-limbs from day 14 post-adjuvant, and the increasing rate in the left hind-limb, relative to day 10 post-adjuvant, was -4.0%, -7.2%, and -6.8% on day 14, day 17, and day 21 post-adjuvant, respectively, while the increasing rate in the right hind-limb, relative to day 10 post-adjuvant, was -6.4%, -9.9%, and -9.7% on day 14, day 17, and day 21 post-adjuvant, respectively.

The group receiving 100 mg/kg of test substance VII-2 showed changes in both the hind-limbs similar to those seen in the corresponding 10-mg/kg group, and a remarkable and significant suppression of the increase in foot volume was found from day 14 post-adjuvant. The increasing rate in foot volume in the left hind-limb, relative to day 10 post-adjuvant, was -5.0%, -7.0%, and -7.0% on day 14, day 17, and day 21 post-adjuvant, respectively, while the increasing rate in foot volume in the right hind-limb, relative to day 10 post-adjuvant, was -6.6%, -6.3%, and -4.8% on day 14, day 17, and day 21 post-adjuvant, respectively: only on day 14 post-adjuvant, the increasing rate in foot volume was found significant.

Table 6a. Percent increase in foot volume relative to pre-adjuvant (left hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | 0.00 ± 0.0 | 6.19 ± 0.4 | 6.33 ± 0.6 | 6.61 ± 0.4 | 7.44 ± 0.4 | 7.85 ± 0.6 | 9.20 ± 0.7 |
| Arthritis (positive) control group | 0.00 ± 0.0 | 12.79 ± 0.5 ## | 12.60 ± 2.0 # | 15.50 ± 1.6 ## | 22.05 ± 2.0 ## | 24.74 ± 1.6 ## | 22.03 ± 1.7 ## |

(continued)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Methotrexate pre-administered group | 0.00 ± 0.0 | 2.29 ± 0.2 | 5.58 ± 2.5 | 7.19 ± 1.8 | 8.39 ± 1.4 ** | 12.44 ± 2.4 | 12.47 ± 2.5 |
| Methotrexate group | 0.00 ± 0.0 | 13.00 ± 0.4 | 15.42 ± 2.5 | 22.63 ± 3.1 | 22.47 ± 3.6 | 28.98 ± 9.6 | 31.36 ± 14.9 |
| Reflumide group | 0.00 ± 0.0 | 13.28 ± 0.4 | 18.56 ± 3.3 | 22.94 ± 3.6 | 20.13 ± 3.2 | 24.18 ± 7.7 | 20.93 ± 7.3 |
| Test substance VII-2 group @ 10 mg/kg | 0.00 ± 0.0 | 12.89 ± 0.3 | 15.37 ± 1.3 | 20.66 ± 1.7 | 15.70 ± 0.9 | 11.93 ± 0.4 | 12.36 ± 0.8 |
| Test substance VII-2 group @ 100 mg/kg | 0.00 ± 0.0 | 13.60 ± 0.5 | 13.58 ± 2.3 | 21.20 ± 3.3 | 15.08 ± 2.8 | 12.49 ± 2.3 | 12.50 ± 2.0 |

#: P < 0.05, ##: P < 0.01, arthritis (positive) control group vs. negative control group

*: P < 0.05, **: P < 0.01, each of the groups vs. arthritis (positive) control group

Table 6b. Percent increase in foot volume relative to pre-adjuvant (right hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | 0.00 ± 0.0 | 5.76 ± 0.2 | 6.84 ± 0.5 | 6.30 ± 0.5 | 6.85 ± 0.4 | 6.96 ± 0.4 | 7.64 ± 0.4 |
| Arthritis (positive) control group | 0.00 ± 0.0 | 11.66 ± 0.5 ## | 21.62 ± 1.8 ## | 30.91 ± 5.5 # | 44.25 ± 6.8 ## | 50.39 ± 12.8 # | 53.04 ± 13.1 # |
| Methotrexate pre-administered group | 0.00 ± 0.0 | 2.30 ± 0.5 | 14.46 ± 2.3 | 15.69 ± 2.2 | 20.20 ± 3.6 * | 32.17 ± 5.7 | 31.22 ± 6.8 |
| Methotrexate group | 0.00 ± 0.0 | 13.20 ± 0.4 | 30.81 ± 4.1 | 55.92 ± 15.4 | 47.20 ± 15.2 | 60.97 ± 23.9 | 57.80 ± 18.3 |
| Reflumide group | 0.00 ± 0.0 | 13.33 ± 0.4 | 26.75 ± 1.4 | 49.75 ± 15.8 | 46.22 ± 10.7 | 43.17 ± 11.2 | 38.93 ± 9.5 |
| Test substance VII-2 group @ 10 mg/kg | 0.00 ± 0.0 | 12.10 ± 0.3 | 28.10 ± 3.4 | 40.36 ± 6.9 | 31.44 ± 7.2 | 26.34 ± 5.6 | 26.57 ± 5.9 |
| Test substance VII-2 group @ 100 mg/kg | 0.00 ± 0.0 | 12.96 ± 0.3 | 28.71 ± 2.3 | 32.68 ± 1.8 | 23.82 ± 2.3 | 24.25 ± 3.3 | 26.31 ± 4.2 |

#: P < 0.05, ##: P < 0.01, arthritis (positive) control group vs. negative control group

*: P < 0.05, **: P < 0.01, each of the groups vs. arthritis (positive) control group

Table 7a. Percent increase in foot volume relative to day 10 post-adjuvant (left hind-limb)

| Group | Day 14 post-adjuvant | Day 17 post-adjuvant | Day 21 post-adjuvant |
|---|---|---|---|
| Negative control group | 0.8 ± 0.7 | 1.2 ± 0.5 | 2.4 ± 0.7 |

(continued)

| Group | Day 14 post-adjuvant | Day 17 post-adjuvant | Day 21 post-adjuvant |
|---|---|---|---|
| Arthritis (positive) control group | $5.7 \pm 1.1$ # | $8.0 \pm 0.7$ ## | $5.7 \pm 1.1$ # |
| Methotrexate pre-administered group | $1.2 \pm 0.5$ | $5.0 \pm 2.4$ | $5.0 \pm 2.8$ |
| Methotrexate group | $-0.1 \pm 1.7$ | $4.9 \pm 6.1$ | $6.6 \pm 10.3$ |
| Reflumide group | $-2.2 \pm 1.6$ | $1.2 \pm 6.3$ | $-1.4 \pm 6.1$ |
| Test substance VII-2 group @ 10 mg/kg | $-4.0 \pm 1.7$ * | $-7.2 \pm 1.3$ * | $-6.8 \pm 1.5$ |
| Test substance VII-2 group @ 100 mg/kg | $-5.0 \pm 0.4$ ** | $-7.0 \pm 1.9$ ** | $-7.0 \pm 1.9$ * |

#: $P < 0.05$, ##: $P < 0.01$, arthritis (positive) control group vs. negative control group

*: $P < 0.05$, **: $P < 0.01$, each of the groups vs. arthritis (positive) control group

Table 7b. Percent increase in foot volume relative to day 10 post-adjuvant (right hind-limb)

| Group | Day 14 post-adjuvant | Day 17 post-adjuvant | Day 21 post-adjuvant |
|---|---|---|---|
| Negative control group | $0.5 \pm 0.5$ | $0.6 \pm 0.6$ | $1.3 \pm 0.8$ |
| Arthritis (positive) control group | $10.2 \pm 1.7$ ## | $14.3 \pm 5.4$ | $16.3 \pm 5.8$ |
| Methotrexate pre-administered group | $3.9 \pm 2.1$ | $14.6 \pm 6.3$ | $13.8 \pm 7.0$ |
| Methotrexate group | $-5.6 \pm 1.9$ * | $2.0 \pm 5.6$ | $1.0 \pm 3.6$ |
| Reflumide group | $-1.3 \pm 2.9$ | $-3.4 \pm 2.8$ | $-5.9 \pm 4.1$ |
| Test substance VII-2 group @ 10 mg/kg | $-6.4 \pm 1.5$ * | $-9.9 \pm 1.4$ * | $-9.7 \pm 2.3$ * |
| Test substance VII-2 group @ 100 mg/kg | $-6.6 \pm 1.4$ ** | $-6.3 \pm 1.7$ | $-4.8 \pm 2.5$ |

#: $P < 0.05$, ##: $P < 0.01$, arthritis (positive) control group vs. negative control group

*: $P < 0.05$, **: $P < 0.01$, each of the groups vs. arthritis (positive) control group

2-3 Scores of Swelling in Knuckle Joints (Tables 8a and 8b)

The negative control group was found to show no abnormalities in both the left and right hind-limbs. In the arthritis control group, both the left and right hind-limbs were found to show changes in their scores on and after day 7 post-adjuvant. The left hind-limb was found to show significant scores increase over time on day 7 and from day 14 to day 21 post-adjuvant, in comparison to the negative control group. The right hind-limb, on the other hand, was found to show significant scores increase on day 14 and day 17 post-adjuvant.

In the methotrexate pre-administered group, both the left and right hind-limbs showed lower scores than those seen in the arthritis control group, but significant changes were not observed at any time points.

In the methotrexate administered group in which methotrexate administration was started on day 10 post-adjuvant, both the left and right hind-limbs showed lower scores than those in the arthritis control group, but significant changes were not observed at any time points. It was only on day 17 post-adjuvant, in both the left and right hind-limbs, that a lower score than that seen in the methotrexate pre-administered group was able to be observed.

In the leflumide group, the scores of 1.4 and 1.6 in the left and right hind-limbs, which were observed before administration on day 10 post-adjuvant, decreased over time down to 0.4 and to 0.6 on day 21 post-adjuvant, respectively.

Also in the group receiving 10 mg/kg of test substance VII-2, the scores of 0.8 and 1.0 in the left and right hind-limbs, which were observed on day 10 post-adjuvant, decreased over time. The left hind-limb showed a score of zero on and after day 17 post-adjuvant, indicating a significant effect of reducing the swelling in the knuckle joints. The right hind-limb, on the other hand, showed a score of 0.2 on day 21 post-adjuvant and reduced the swelling in the knuckle joints.

In the group receiving 100 mg/kg of test substance VII-2, the scores of 0.6 and 1.0 in the left and right hind-limbs, which were observed on day 10 post-adjuvant, decreased over time. The left hind-limb showed a score of zero on and after day 17 post-adjuvant, indicating a significant effect of reducing the swelling in the knuckle joints. The right hind-limb, on the other hand, showed a score of 0.2 on day 21 post-adjuvant and reduced the swelling in the knuckle joints.

Table 8a. Scores of swelling in knuckle joints (left hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | 0.0 ± 0.0 | 0.0 ± 0.0 | 0.0 ± 0.0 | 0.0 ± 0.0 | 0.0 ± 0.0 | 0.0 ± 0.0 | 0.0 ± 0.0 |
| Arthritis (positive) control group | 0.0 ± 0.0 | 0.0 ± 0.0 | 0.8 ± 0.2 # | 0.4 ± 0.2 | 1.0 ± 0.0 ## | 1.2 ± 0.2 ## | 1.0 ± 0.0 ## |
| Methotrexate pre-administered group | 0.0 ± 0.0 | 0.0 ± 0.0 | 0.0 ± 0.0 | 0.2 ± 0.2 | 0.4 ± 0.2 * | 0.6 ± 0.2 | 0.4 ± 0.2 |
| Methotrexate group | 0.0 ± 0.0 | 0.0 ± 0.0 | 0.4 ± 0.2 | 0.6 ± 0.2 | 0.8 ± 0.2 | 0.4 ± 0.2 | 0.6 ± 0.6 |
| Reflumide group | 0.0 ± 0.0 | 0.0 ± 0.0 | 0.4 ± 0.2 | 1.4 ± 0.4 | 0.6 ± 0.4 | 0.6 ± 0.4 | 0.4 ± 0.4 |
| Test substance VII-2 group @ 10 mg/kg | 0.0 ± 0.0 | 0.0 ± 0.0 | 0.4 ± 0.2 | 0.8 ± 0.2 | 0.0 ± 0.0 | 0.0 ± 0.0 ** | 0.0 ± 0.0 ** |
| Test substance VII-2 group @ 100 mg/kg | 0.0 ± 0.0 | 0.0 ± 0.0 | 0.6 ± 0.2 | 0.6 ± 0.2 | 0.4 ± 0.2 | 0.0 ± 0.0 ** | 0.0 ± 0.0 ** |

#: $P < 0.05$, ##: $P < 0.01$, arthritis (positive) control group vs. negative control group
*: $P < 0.05$, **: $P < 0.01$, each of the groups vs. arthritis (positive) control group

Table 8b. Scores of swelling in knuckle joints (right hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | 0.0 ± 0.0 | 0.0 ± 0.0 | 0.0 ± 0.0 | 0.0 ± 0.0 | 0.0 ± 0.0 | 0.0 ± 0.0 | 0.0 ± 0.0 |
| Arthritis (positive) control group | 0.0 ± 0.0 | 0.0 ± 0.0 | 0.6 ± 0.2 | 0.6 ± 0.2 | 1.4 ± 0.2 ## | 1.8 ± 0.6 # | 1.6 ± 0.6 |
| Methotrexate pre-administered group | 0.0 ± 0.0 | 0.0 ± 0.0 | 0.4 ± 0.2 | 0.2 ± 0.2 | 0.8 ± 0.2 | 1.0 ± 0.3 | 0.8 ± 0.2 |
| Methotrexate group | 0.0 ± 0.0 | 0.0 ± 0.0 | 0.6 ± 0.2 | 1.6 ± 0.4 | 1.2 ± 0.2 | 0.6 ± 0.2 | 0.8 ± 0.6 |
| Reflumide group | 0.0 ± 0.0 | 0.0 ± 0.0 | 0.2 ± 0.2 | 1.6 ± 0.6 | 1.4 ± 0.4 | 0.0 ± 0.0 | 0.6 ± 0.6 |
| Test substance VII-2 group @ 10 mg/kg | 0.0 ± 0.0 | 0.0 ± 0.0 | 0.4 ± 0.2 | 1.0 ± 0.0 | 0.8 ± 0.2 | 0.4 ± 0.2 | 0.2 ± 0.2 |
| Test substance VII-2 group @ 100 mg/kg | 0.0 ± 0.0 | 0.0 ± 0.0 | 0.4 ± 0.2 | 1.0 ± 0.0 | 1.0 ± 0.0 | 0.4 ± 0.2 | 0.2 ± 0.2 |

# : $P < 0.05$, ## : $P < 0.01$, arthritis (positive) control group vs. negative control group
* : $P < 0.05$, ** : $P < 0.01$, each of the groups vs. arthritis (positive) control group

2-4 Scores of Swelling in Insteps (Tables 9a and 9b)

The negative control group was found to show no abnormalities in both the left and right hind-limbs. In the arthritis control group, both the left and right hind-limbs were found to show changes in their scores on and after day 7 post-adjuvant. The left hind-limb was found to show significant score increases over time from day 10 to day 21 post-adjuvant, in comparison to the negative control group. The right hind-limb, on the other hand, was found to show significant score increases on and after day 7 post-adjuvant.

In the methotrexate pre-administered group, both the left and right hind-limbs showed lower scores than those seen in the arthritis control group. The left hind-limb was found to show significant score decreases on day 10 and day 14 post-adjuvant, in comparison to the arthritis control group. The right hind-limb, on the other hand, was found to show significant score decreases from day 7 to day 14 post-adjuvant. In both the hind-limbs, the scores rapidly increased on day 17 and day 21 post--adjuvant.

In the Methotrexate administered group in which methotrexate administration was started on day 10 post-adjuvant, no differences were found relative to the arthritis control group, except that a tendency of score improvement was shown only in the left hind-limb on day 14 post-adjuvant.

In the leflumide group, the left hind-limb was found to show, on and after day 14 post-adjuvant, scores lower than the score of 0.6 on day 10 post-adjuvant, while the right hind-limb has such scores that the score on day 10 post-adjuvant did'not change to worse values on and after day 14 post-adjuvant.

In the group receiving 10 mg/kg of test substance VII-2, the left hind-limb had a low score of 0.2 on day 10 post-adjuvant, but a significant effect was showed in which the score was zero on and after day 14 post-adjuvant. The right hind-limb, on the other hand, had a score of 0.8 on day 10 post-adjuvant, which did not change to worse values on and after day 14 post-adjuvant.

In the group receiving 100 mg/kg of test substance VII-2, although the left hind-limb had a score of zero on day 10 post-adjuvant, one animal with instep swelling was observed on day 21 post-adjuvant, whereby a score of 0.2 was obtained. In the right hind-limb, the score of 1.0 on day 10 post-adjuvant did not change on and after day 14 post-adjuvant and the scores until day 17 post-adjuvant were significant.

Table 9a. Scores of swelling in the instep (left hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | $0.0 \pm 0.0$ | $0.0 \pm 0.0$ | $0.0 \pm 0.0$ | $0.0 \pm 0.0$ | $0.0 \pm 0.0$ | $0.0 \pm 0.0$ | $0.0 \pm 0.0$ |
| Arthritis (positive) control group | $0.0 \pm 0.0$ | $0.0 \pm 0.0$ | $0.4 \pm 0.2$ | $1.0 \pm 0.0$ ## | $1.0 \pm 0.0$ ## | $1.0 \pm 0.0$ ## | $1.0 \pm 0.0$ ## |
| Methotrexate pre-administered group | $0.0 \pm 0.0$ | $0.0 \pm 0.0$ | $0.0 \pm 0.0$ | $0.0 \pm 0.0$ ** | $0.0 \pm 0.0$ ** | $0.4 \pm 0.2$ | $0.2 \pm 0.2$ * |
| Methotrexate group | $0.0 \pm 0.0$ | $0.0 \pm 0.0$ | $0.0 \pm 0.0$ | $0.6 \pm 0.2$ | $0.2 \pm 0.2$ | $0.8 \pm 0.6$ | $0.6 \pm 0.6$ |
| Reflumide group | $0.0 \pm 0.0$ | $0.0 \pm 0.0$ | $0.4 \pm 0.2$ | $0.6 \pm 0.4$ | $0.2 \pm 0.2$ | $0.2 \pm 0.2$ | $0.4 \pm 0.4$ |
| Test substance VII-2 group @ 10 mg/kg | $0.0 \pm 0.0$ | $0.0 \pm 0.0$ | $0.2 \pm 0.2$ | $0.2 \pm 0.2$ | $0.0 \pm 0.0$ ** | $0.0 \pm 0.0$ ** | $0.0. \pm 0.0$ ** |
| Test substance VII-2 group @ 100 mg/kg | $0.0 \pm 0.0$ | $0.0 \pm 0.0$ | $0.4 \pm 0.2$ | $0.0 \pm 0.0$ | $0.0 \pm 0.0$ ** | $0.0 \pm 0.0$ ** | $0.2 \pm 0.2$ * |

\#: P < 0.05, ## : P < 0.01, arthritis (positive) control group vs. negative control group
\* : P < 0.05, ** : P < 0.01, each of the groups vs. arthritis (positive) control group

Table 9b. Scores of swelling in the instep (right hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | 0.0 ± 0.0 | 0.0 ± 0.0 | 0.0 ± 0.0 | 0.0 ± 0.0 | 0.0 ± 0.0 | 0.0 ± 0.0 | 0.0 ± 0.0 |
| Arthritis (positive) control group | 0.0 ± 0.0 | 0.0 ± 0.0 | 1.0 ± 0.0 ## | 2.2 ± 0.2 ## | 2-2 ± 0.2 ## | 2.4 ± 0.4 ## | 1.8 ± 0.4 ## |
| Methotrexate pre-administered group | 0.0 ± 0.0 | 0.0 ± 0.0 | 0.0 ± 0.0 | 0.4 ± 0.2 ** | 0.8 ± 0.2 ** | 1.4 ± 0.2 | 1.2 ± 0.4 |
| Methotrexate group | 0.0 ± 0.0 | 0.0 ± 0.0 | 1.2 ± 0.2 | 1.4 ± 0.4 | 1.4 ± 0.4 | 2.0 ± 0.4 | 2.2 ± 0.6 |
| Reflumide group | 0.0 ± 0.0 | 0.0 ± 0.0 | 1.0 ± 0.0 | 1.4 ± 0.4 | 1.2 ± 0.2 | 1.2 ± 0.2 | 1.4 ± 0.4 |
| Test substance VII-2 group @ 10 mg/kg | 0.0 ± 0.0 | 0.0 ± 0.0 | 0.4 ± 0.2 | 0.8 ± 0.2 ** | 0.8 ± 0.2 ** | 0.6 ± 0.2 ** | 0.8 ± 0.2 |

#: P < 0.05, ##: P < 0.01, arthritis (positive) control group vs. negative control group
* : P < 0.05, ** : P < 0.01, each of the groups vs. arthritis (positive) control group

2-5 Scores of Redness (Tables 10a and 10b)

The negative control group was found to show no abnormalities in both the left and right hind-limbs. In the arthritis control group, the left hind-limb was found to show changes in its score on and after day 10 post-adjuvant and to have a significant score increase on day 14 post-adjuvant in comparison to the negative control group. The right hind-limb, on the other hand, was found to show changes in its score on and after day 7 post-adjuvant and to have significant score increases on day 10 and day 14 post-adjuvant.

In the methotrexate pre-administered group, both the left and right hind-limbs showed lower scores than those seen in the arthritis control group, while it was found that the left hind-limb showed a significant score decrease on day 14 post-adjuvant and the right hind-limb showed a significant score decrease on day 10 and day 14 post-adjuvant, in comparison to the arthritis control group.

In the methotrexate administered group in which methotrexate administration was started on day 10 post-adjuvant, it was only on day 14 post-adjuvant that the left hind-limb showed a lower score than that seen in the arthritis control group, while the right hind-limb showed a lower score than that seen in the arthritis control group, on and after day 14 post-adjuvant, but these score decreases in both the hind-limbs were not significant.

In the leflumide group, there was shown a tendency that the scores of 0.8 and 1.2 in the left and right hind-limbs, which were observed before administration on day 10 post-adjuvant, slightly decreased in the left hind-limb, but the score in the right hind-limb on day 21 post-adjuvant rose to 1.4.

In the group receiving 10 mg/kg of test substance VII-2, a significant effect of reducing the redness was shown in which the score of 0.2 in the left hind-limb on day 10 post-adjuvant decreased to zero on and after day 14 post-adjuvant. In the right hind-limb, on the other hand, the score did not change until day 17 post-adjuvant and slightly decrease to 0.6 on day 21 post-adjuvant.

In the group receiving 100 mg/kg of test substance VII-2, although the left hind-limb had a score of zero on day 10 post-adjuvant, one animal with redness was observed on day 17 and on day 21 post-adjuvant, respectively, whereby the score rose to 0.2 in each case. In the right hind-limb, the score of 1.0 on day 10 post-adjuvant did not become worse on and after day 14 post-adjuvant.

Table 10a. Redness scores (left hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | 0.0 ± 0.0 | 0.0 ± 0.0 | 0.0 ± 0.0 | 0.0 ± 0.0 | 0.0 ± 0.0 | 0.0 ± 0.0 | 0.0 ± 0.0 |
| Arthritis (positive) control group | 0.0 ± 0.0 | 0.0 ± 0.0 | 0.0 ± 0.0 | 0.4 ± 0.2 | 0.8 ± 0.2 # | 0.6 ± 0.2 | 0.6 ± 0.2 |
| Methotrexate pre-administered group | 0.0 ± 0.0 | 0.0 ± 0.0 | 0.0 ± 0.0 | 0.0 ± 0.0 | 0.0 ± 0.0 ** | 0.4 ± 0.4 | 0.2 ± 0.2 |
| Methotrexate group | 0.0 ± 0.0 | 0.0 ± 0.0 | 0.0 ± 0.0 | 0.4 ± 0.6 | 0.2 ± 0.2 | 0.6 ± 0.4 | 0.6 ± 0.6 |
| Reflumide group | 0.0 ± 0.0 | 0.0 ± 0.0 | 0.4 ± 0.2 | 0.8 ± 0.4 | 0.4 ± 0.2 | 0.6 ± 0.6 | 0.6 ± 0.6 |
| Test substance VII-2 group @ 10 mg/kg | 0.0 ± 0.0 | 0.0 ± 0.0 | 0.0 ± 0.0 | 0.2 ± 0.2 | 0.0 ± 0.0 ** | 0.0 ± 0.0 | 0.0 ± 0.0 |
| Test substance VII-2 group @ 100 mg/kg | 0.0 ± 0.0 | 0.0 ± 0.0 | 0.0 ± 0.0 | 0.0 ± 0.0 | 0.0 ± 0.0 ** | 0.2 ± 0.2 | 0.2 ± 0.2 |

\#: $P < 0.05$, \#\#: $P < 0.01$, arthritis (positive) control group vs. negative control group
\* : $P < 0.05$, \*\* : $P < 0.01$, each of the groups vs. arthritis (positive) control group

Table 10b. Redness scores (right hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | 0.0 ± 0.0 | 0.0 ± 0.0 | 0.0 ± 0.0 | 0.0 ± 0.0 | 0.0 ± 0.0 | 0.0 ± 0.0 | 0.0 ± 0.0 |
| Arthritis (positive) control group | 0.0 ± 0.0 | 0.0 ± 0.0 | 0.6 ± 0.2 | 1.2 ± 0.2 ## | 1.4 ± 0.4 # | 1.6 ± 0.6 | 1.6 ± 0.6 |
| Methotrexate pre-administered group | 0.0 ± 0.0 | ± 0.0 ± 0.0 | 0.2 ± 0.2 | 0.0 ± 0.0 ** | 0.2 ± 0.2 ** | 0.8 ± 0.4 | ± 0.8 ± 0.2 |
| Methotrexate group | 0.0 ± 0.0 | 0.0 ± 0.0 | 0.4 ± 0.2 | 1.4 ± 0.4 | 1.0 ± 0.3 | 1.2 ± 0.2 | 1.2 ± 0.5 |
| Reflumide group | 0.0 ± 0.0 | 0.0 ± 0.0 | 0.6 ± 0.2 | 1.2 ± 0.2 | 1.2 ± 0.2 | 0.8 ± 0.2 | 1.4 ± 0.4 |
| Test substance VII-2 group @ 10 mg/kg | 0.0 ± 0.0 | 0.0 ± 0.0 | 0.2 ± 0.2 | 1.0 ± 0.0 | 1.0 ± 0.0 | 1.0 ± 0.0 | 0.6 ± 0.2 |
| Test substance VII-2 group @ 100 mg/kg | 0.0 ± 0.0 | 0.0 ± 0.0 | 0.8 ± 0.2 | 1.0 ± 0.0 | 1.0 ± 0.0 | 1.0 ± 0.3 | 1.0 ± 0.0 |

\#: $P < 0.05$, \#\# : $P < 0.01$, arthritis (positive) control group vs. negative control group
\* : $P < 0.05$, \*\* : $P < 0.01$, each of the groups vs. arthritis (positive) control group

2-6 Total scores (Tables 11a and 11b)

In the negative control group, both the left and right hind-limbs were found to show no abnormalities. In the arthritis control group, both the left and right hind-limbs were found to show changes in their scores and to have a significant score increase in comparison to the negative control group, on and after day 7 post-adjuvant. The left hind-limb reached its highest score of 2.8 on day 14 post-adjuvant, which after that, remained almost unchanged. The right hind-limb, on the other hand, reached its highest score of 5.8 on day 17 post-adjuvant.

In the methotrexate pre-administered group, both the left and right hind-limbs showed lower scores than those seen in the arthritis control group, while it was found that the left hind-limb showed significant score decreases on day 10 and day 14 post-adjuvant and the right hind-limb showed significant score decreases on day 7 and day 10 post-adjuvant, in comparison to the arthritis control group. However, the left hind-limb showed high scores of 1.4 and 0.8 on day 17 and day 21 post-adjuvant, respectively, and these scores were not significant. Also, the right hind-limb showed high scores of 1.8, 3.2, and 2.8 on day 14, day 17, and day 21 post-adjuvant, respectively, and these scores were not significant.

In the methotrexate administration group in which methotrexate administration was started on day 10 post-adjuvant, the left hind-limb showed high scores of 1.2, 1.8, and 1.8 on day 14, day 17, and day 21 post-adjuvant, respectively, and these scores were not significant. Also, the right hind-limb showed high scores of 3.6, 3.8, and 4.2 on day 14, day 17, and day 21 post-adjuvant, respectively, and these scores were not significant.

In the leflumide group, there was shown a tendency that the scores of 2.8 and 4.2 in the left and right hind-limbs, which were observed before administration on day 10 post-adjuvant, decreased to half or less on and after day 14 post-adjuvant, and such decreases were not significant. The right hind-limb, on the other hand, showed a significantly decreased score of 2.0 on day 17 post-adjuvant.

In the group receiving 10 mg/kg of test substance VII-2, both the hind-limbs showed significant score decreases on and after day 14 post-adjuvant.

In the group receiving 100 mg/kg of test substance VII-2, the left hind-limb showed significant score decreases on and after day 14 post-adjuvant, while in the right hind-limb, a score decreasing effect was showed on and after day 14 post-adjuvant and the scores on day 14 and 21 post-adjuvant were significant.

Table 11a. Total scores (left hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | $0.0 \pm 0.0$ | $0.0 \pm 0.0$ | $0.0 \pm 0.0$ | $0.0 \pm 0.0$ | $0.0 \pm 0.0$ | $0.0 \pm 0.0$ | $0.0 \pm 0.0$ |
| Arthritis (positive) control group | $0.0 \pm 0.0$ | $0.0 \pm 0.0$ | $1.2 \pm 0.4$ # | $1.8 \pm 0.2$ ## | $2.8 \pm 0.2$ ## | $2.8 \pm 0.4$ ## | $2.6 \pm 0.2$ ## |
| Methotrexate pre-administered group | $0.0 \pm 0.0$ | $0.0 \pm 0.0$ | $0.0 \pm 0.0$ | $0.2 \pm 0.2$ ** | $0.4 \pm 0.2$ * | $1.4 \pm 0.7$ | $0.8 \pm 0.6$ |
| Methotrexate group | $0.0 \pm 0.0$ | $0.0 \pm 0.0$ | $0.4 \pm 0.5$ | $1.6 \pm 0.5$ | $1.2 \pm 0.6$ | $1.8 \pm 1.4$ | $1.8 \pm 2.3$ |
| Reflumide group | $0.0 \pm 0.0$ | $0.0 \pm 0.0$ | $1.2 \pm 1.6$ | $2.8 \pm 1.4$ | $1.2 \pm 1.0$ | $1.4 \pm 1.5$ | $1.4 \pm 1.8$ |
| Test substance VII-2 group @ 10 mg/kg | $0.0 \pm 0.0$ | $0.0 \pm 0.0$ | $0.6 \pm 0.4$ | $1.2 \pm 0.2$ | $0.0 \pm 0.0$ ** | $0.0 \pm 0.0$ ** | $0.0 \pm 0.0$ ** |
| Test substance VII-2 group @ 100 mg/kg | $0.0 \pm 0.0$ | $0.0 \pm 0.0$ | $1.0 \pm 0.4$ | $0.6 \pm 0.2$ | $0.4 \pm 0.2$ * | $0.2 \pm 0.2$ * | $0.4 \pm 0.4$ * |

# : P < 0.05, ##: P < 0.01, arthritis (positive) control group vs. negative control group

* : P < 0.05, ** : P < 0.01, each of the groups vs. arthritis (positive) control group

Table 11b. Total scores (right hind-limb)

| Group | Day 0 post-adjuvant | Day 3 post-adjuvant | Day 7 post-adjuvant | Day 10 post-adjuvant | Day 14 post-adjuvant | Day 17 post-adjuvant | Day 21 post-adjuvant |
|---|---|---|---|---|---|---|---|
| Negative control group | 0.0 ± 0.0 | 0.0 ± 0.0 | 0.0 ± 0.0 | 0.0 ± 0.0 | 0.0 ± 0.0 | 0.0 ± 0.0 | 0.0 ± 0.0 |
| Arthritis (positive) control group | 0.0 ± 0.0 | 0.0 ± 0.0 | 2.2 ± 0.4 ## | 4.0 ± 0.5 ## | 5.0 ± 0.8 ## | 5.8 ± 1.6 # | 5.0 ± 1.5 # |
| Methotrexate pre-administered group | 0.0 ± 0.0 | 0.0 ± 0.0 | 0.6 ± 0.4 ** | 0.6 ± 0.4 ** | 1.8 ± 0.2 * | 3.2 ± 0.8 | 2.8 ± 0.6 |
| Methotrexate group | 0.0 ± 0.0 | 0.0 ± 0.0 | 2.2 ± 0.8 | 4.4 ± 1.5 | 3.6 ± 1.1 | 3.8 ± 0.9 | 4.2 ± 2.0 |
| Reflumide group | 0.0 ± 0.0 | 0.0 ± 0.0 | 1.8 ± 0.5 | 4.2 ± 1.5 | 3.8 ± 1.0 | 2.0 ± 0.0 ** | 3.4 ± 1.8 |
| Test substance VII-2 group @ 10 mg/kg | 0.0 ± 0.0 | 0.0 ± 0.0 | 1.0 ± 0.5 | 2.8 ± 0.2 | 2.6 ± 0.2 * | 2.0 ± 0.4 * | 1.6 ± 0.2 ** |
| Test substance VII-2 group @ 100 mg/kg | 0.0 ± 0.0 | 0.0 ± 0.0 | 2.0 ± 0.5 | 3.0 ± 0.0 | 2.6 ± 0.2 * | 2.2 ± 0.6 | 2.2 ± 0.4 * |

#: $P < 0.05$, ## : $P < 0.01$, arthritis (positive) control group vs. negative control group

*: $P < 0.05$, ** : $P < 0.01$, each of the groups vs. arthritis (positive) control group

2-7 IL-1β and TNF-α in Plasma (Table 12)

On day 21 post-adjuvant, the animals were subjected to autopsy, and plasma was prepared to measure IL-1β and TNF-α concentrations in plasma. In all the groups for which measurements were made, the concentration of TNF-α was below the detection limit (8 pg/mL).

Regarding the concentration of IL-1β, the negative control group had a value of 20.6 pg/mL and the arthritis control group had a lower value of 15.1 pg/mL. The concentration of IL-1β was 18.4 and 22.6 pg/mL in the methotrexate pre-administered group and in the methotrexate administered group in which methotrexate administration was started on day 10 post-adjuvant, respectively.

The leflumide group showed a low value of 13.0 pg/mL.

For the group receiving test substance VII-2, the concentration of IL-1β was 10.5 and 13.9 pg/mL in the 10- and 100-mg/mL groups, respectively.

Table 12. Concentrations of IL-1β and TNF-α in plasma

| Group | 1L-1β (pg/mL) | TNF-α |
|---|---|---|
| Negative control group | 20.6 ± 4.6 | N.D |
| Arthritis (positive) control group | 15.1 ± 3.8 | N.D |
| Methotrexate pre- administered group | 18.4 ± 4.2 | N.D |
| Methotrexate group | 22.6 ± 11.3 | N.D |
| Reflumide group | 13.0 ± 2.5 | N.D |
| Test substance VII-2 group @ 10 mg/kg | 10.5 ± 4.6 | N.D |
| Test substance VII-2 group @ 100 mg/kg | 13.9 ± 3.7 | N.D |

N.D.: below detection limit

3. Discussion

In order to ascertain the anti-inflammatory effect of test substance VII-2, the examination was carried out by comparing substance VII-2 with the positive control agents, using as markers arthritis scores, amounts of edema in the hind-limbs, and amounts of TNF-$\alpha$ and IL-1$\beta$ in the plasma from animals 21 days after adjuvant injection, in an adjuvant arthritis model using rats.

The negative control group was found to show normal increases in foot volume with growth in both the left and right hind-limbs and the increasing rate in foot volume on day 21 post-adjuvant was 9.20% for the left hind-limb and 7.65% for the right hind-limb.

The arthritis control group was found to show remarkable increases in foot volume caused by the adjuvant in both the left and right hind-limbs, and had significant increases in foot volume over time in both the hind-limbs on and after day 3. In addition, increases in all the scores were found also in the left hind-limb on day 10 post-adjuvant, and outcomes were achieved which well reproduced not only the increase in hind-limb volume, but also rheumatoid inflammation associated with immune reactions.

The methotrexate pre-administered group in which methotrexate had been administered before adjuvant injection on day 0 post-adjuvant showed changes in the left hind-limb similar to those seen in the negative control group until day 14 post-adjuvant. Although a significant effect of suppressing the increase in foot volume was found on day 14 post-adjuvant, the foot volume and the respective scores rapidly increased on days 17 and 21 post-adjuvant. Outcomes were achieved which well reproduced the results that the inflammation are not completely suppressed by methotrexate, as usually observed in the present model and collagen arthritis models.

In the group receiving therapeutic administrations of methotrexate in which methotrexate administration was started on day 10 post-adjuvant, the hind-limb volume turned to increase on and after day 17 post-adjuvant and was comparable to that in the arthritis control group on day 21 post-adjuvant. In addition, the score shown by methotrexate was the highest in those shown by the test substances used and was almost close to the value seen in the arthritis control group.

Therefore, it is believed that a data that the therapeutic administrations of methotrexate are ineffective was able to be reproduced.

The leflumide group was not found to show large changes in the increasing rate in foot volume in the left and right hind-limbs. However, on day 21 post-adjuvant, relative to day 10 post-adjuvant, only a suppression of the increase in foot volume was shown, becoming slightly negative values for both the hind-limbs, and a strong effect of reducing the amount of edema in the limbs was not ascertained. In the right hind-limb, it was the total score on day 17 post-adjuvant that was significant in the scores. However, an evaluation of having become worse at the time of day 21 post-adjuvant was made only for the redness score in the right hind-limb, which was at such an extent that the score of 1.2 in the right hind-limb, observed before administration on day 10 post-adjuvant, increased to 1.4, and all the other scores showed decreasing trends. Therefore, leflumide would have a weak effect of suppressing edema in this examination, but exhibit a sufficient drug efficacy.

The groups receiving 10 and 100 mg/kg of test substance VII-2 showed a remarkable and significant suppression of the increase in foot volume in both the hind-limbs from day 14 post-adjuvant, and the suppression effects seen in both these groups were similar and comparative anti-inflammatory effects. Therefore, the administration of test substance VII-2 at 10 mg/kg would have a sufficient anti-inflammatory effect. Particularly, from the tendency to suppress the increase in foot volume in the right hind-limb, test substance VII-2 would have an anti-inflammatory effect on complex cases of acute inflammation and immune inflammation represented in limbs to which an adjuvant and a drug have been administered.

In the case of swelling in knuckle joints as a marker of joint destruction, the suppression effect which was exhibited at 10 and 100 mg/kg of test substance VII-2 was strong, and suppression effects that were equal to or higher than those in the methotrexate pre-administered, methotrexate administered, and leflumide administered groups were achieved on and after day 14 post-adjuvant. Also in the case of the effect of reducing edema represented by instep swelling, the effect exhibited by test substance VII-2 was strong, and suppression effects that were equal to or higher than those in the methotrexate pre-administered, methotrexate administered, and leflumide administered groups were achieved on and after day 14 post-adjuvant.

Except for the negative control group, the groups in an increasing order of the IL-1$\beta$ concentration on day 21 post-adjuvant are: the 10-mg/kg test substance VII-2 group, the leflumide group, the 100-mg/kg test substance VII-2 group, the arthritis control group, the methotrexate pre-administered group, and the methotrexate group, reflecting directly the situation of exhibiting the anti-inflammatory effect on day 21 post-adjuvant in this examination. In contrast, the concentration of TNF-$\alpha$ in plasma on day 21 post-adjuvant was below the detection limit (8 pg/mL) in all the groups.

Summarizing these results, test substance VII-2, when administered at 10 mg/kg, was demonstrated to exhibit a strong anti-inflammatory effect similar to that exhibited by its administration at 100 mg/kg. This effect is likely to be due to the contribution of high solubility of test substance VII-2 in water. Test substance VII-2 was also demonstrated to exhibit an arthritis suppressing effect equal to or higher than that in the methotrexate pre-administered group, on and after day 14 post-adjuvant. In addition, test substance VII-2 was found to exhibit a stronger arthritis suppressing effect than that of leflumide, which is an antirheumatic drug.

From these examination results, it is concluded that test substance VII-2 has an arthritis suppressing effect equal to or higher than those of methotrexate and leflumide and is effective particularly against immune inflammation, such as rheumatism.

Note that methotrexate is known to cause serious adverse effects even when used in very small amounts. The peptide of the present invention, on the other hand, is derived from the natural substance and did not display any detectable adverse effects in the above-described examinations. Therefore, it can be said that the peptide of the present invention is more beneficial, relative to conventionally used pharmaceuticals, also in the viewpoint of safety. Moreover, test substance VII-2, even when administered at a dose of 10 mg/kg, was demonstrated to have an arthritis suppressing effect equivalent to that exhibited when administered at a dose of 100 mg/kg. These results would be likely to be not only due to high effectiveness of test substance VII-2 itself, but also due to its high water solubility

Example 6

Study of Immune-Complex Disease Suppressing Effects of Peptide of The Present Invention Immune-Complex Disease Suppressing Effects of Peptide of The Present Invention in Type III Allergic (Arthus) Reaction-Induced Rat Model

1. Materials and Methods

1-1 Animals and Experimental Procedures

Using forty-two 7-week-old male Slc:Wistar rats, examinations were made on test substance VII-2, with each of intraperitoneal administration and intravenous administration into tail vain. Acclimation was carried out by giving normal solid chow CRF-1 for a period of 6 days or longer. On the day prior to administration, the back of each animal was shaved. On the day of administration, a mixed solution of egg-white Ovalbumin (MP Biomedicals) (hereinafter referred to as "OVA") and Evans blue dye was administered into the tail vein. A solution of the test substance was administered intraperitoneally or into the tail vein 30 minutes or 50 minutes after the administration of the mixed solution of OVA and Evans blue dye, respectively. For intracutaneous administration of a solution of anti-OVA (Nordic immunological Laboratories), the solution was intracutaneously administered in the back of each animal at a dose of 0.1 ml/site 30 minutes or 10 minutes after the administration of the test substance in the case of its intraperitoneal or intravenous administration, respectively, so as to generate a local Arthus reaction. After the reaction was allowed to be generated for 4 hours, the animals were euthanized, the blood was removed thoroughly, and then the dorsal skin was exfoliated. The Arthus reaction sites on the exfoliated skin were punched out, and the Evans blue dye was extracted from the punched skin overnight. The amount of dye leakage was quantified by measuring absorbance for the Evans blue dye with a spectrophotometer and using a calibration curve.

1-2 Control and Test Substances

A negative control substance used physiological saline, and a positive control used a solution of hydrocortisone. The hydrocortisone solution was prepared by placing a predetermined amount of hydrocortisone into a mortar and grinding it with a pestle, followed by adding physiological saline, to make a solution of a final concentration of 10 mg/mL, which was used as a solution for administration of the positive control substance.

The test substance solution was prepared as follows.

A predetermined amount of test substance VII-2 was dissolved in physiological saline to make a 20 mg/ml solution, which was used as an administration solution for a group receiving 100-mg/kg intraperitoneal administration. An administration solution for a group receiving 10-mg/kg intraperitoneal administration was prepared as a 2 mg/mL solution by 10-times dilution of the 20 mg/ml solution with physiological saline. An administration solution for a group receiving 100-mg/kg intravenous administration into tail vain was prepared by dissolving a predetermined amount of test substance VII-2 in physiological saline to make a 50 mg/mL solution, which was used as an administration solution.

1-3 Arthus Reaction

The mixed solution of OVA and Evans blue dye was administered into the tail vein at a volume of 2 ml/kg. Each of the peptide solutions was administered intraperitoneally or into the tail vein 30 minutes or 50 minutes after the administration of the mixed solution of OVA and Evans blue dye, respectively. For intracutaneous administration of a solution of anti-OVA, the solution was intracutaneously administered in the back of each animal at a dose of 0.1 mL/site 30 minutes or 10 minutes after the administration of the test substance in the case of its intraperitoneal or intravenous administration, respectively, so as to generate a local Arthus reaction. Intracutaneous administration was conducted at 2 sites for PBS (PBS(1) and PBS(2)) and at 2 sites for the anti-OVA solution (anti-OVA(1) and anti-OVA(2)) per animal. After the reaction was allowed to be generated for 4 hours, the animals were euthanized by decapitation under ether anesthesia, the blood was removed thoroughly, and then the dorsal skin was exfoliated. The Arthus reaction sites on the exfoliated skin were punched out and subjected to dye extraction.

1-4 Dye Extraction and Measurement

Several cuttings were made in the skin strips that had been punched out with a punch. The strips were then immersed in 2 ml of a dye extraction solution and stirred with shaking for 10 minutes. Subsequently, the strips were left standing overnight at room temperature. After being left to stand overnight, the strips were stirred again with shaking for 10 minutes, followed by centrifugation (1500 rpm, 15 minutes). The supernatant was used as a sample for dye measurement. A UV-1600 (Shimadzu Corporation) was used for dye measurement, which was performed at a wavelength of OD 620 nm. The amount of dye leakage was calculated from a calibration curve of known amounts of the dye.

1-5 Examination Groups

Examination groups were as indicated below:

Table 13

| (1) Intraperitoneal administration group | Negative control substance administered group |
| --- | --- |
| | 10-mg/kg test substance VII-2 administered group |
| | 100-mg/kg test substance VII-2 administered group |
| | 50-mg/kg positive substance administered group |
| (2) Intravenous administration into tail vain group | Negative control substance administered group |
| | 100-mg/kg test substance VII-2 administered group |

1-6 Data Processing and Statistical Analysis

The mean value (mean) $\pm$ standard deviation (SD) for each of the groups was calculated with respect to body-weight measurements and amounts of dye leakage. For amounts of dye leakage, the value for each of the animals was determined by subtracting the mean of the values obtained for the two sites of PBS administration from the mean of the values obtained for the two sites of administration of the anti-OVA solution. Statistical analysis was performed on the thus obtained amounts of dye leakage, as follows.
In the examination by intraperitoneal administration of the test substance, Bartlett's test of homogeneity of variances was performed between each of the groups. Since a result was obtained that there was no difference in the variances, Dunnett's multiple comparison test was used to test the difference in the mean values between the negative control group and each of the other groups. F testing was performed between the negative and positive control groups. Since a result was obtained that there was a difference in the variances, Aspin-Welch t-test was used to test the difference in the mean values between the two groups.
In the examination by intravenous administration into tail vain of the test substance, F testing was performed between the negative control group and the test substance administered group. Since a result was obtained that there was no difference in the variances, Student t-test was used to test the difference in the mean values between the two groups. The level of significance was set at 5% in the Bartlett's test of homogeneity of variances and at 5% on both sides in the others.

2. Results

2-1 Body Weight Changes and General Conditions of Animals

Throughout the period of these examinations, all the animals in the groups were found to show a steady increase in body weight. There were no significant differences in changes of body weight between the groups. In addition, there were neither observed any abnormalities in general conditions nor any symptoms which seemed to be due to adverse effects for all the animals throughout the period of these examinations.

2-2 Results of Measurement of Amounts of Dye Leakage and Statistical Analysis

The concentration of leakage dye ($\mu$g/mL) was calculated for each of the sites on the respective animals where the Arthus reaction was allowed to be generated (intradermal administration sites). The amount of leakage dye per animal ($\mu$g/animal) was calculated by substituting the concentration of leakage dye calculated for each of the sites into the following formula:

```
*Calculating formula: [((anti-OVA(1) + anti-
OVA(2))/2) - ((PBS(1) + PBS(2))/2)] × 2
```

2-2-1 Results of Intraperitoneal Administration Examination (see, Fig. 16(a))

The mean value (mean) $\pm$ standard deviation (SD) in each of the groups was: 11.099 $\pm$ 3.071 $\mu$g/animal for the negative control substance administered group; 8.705 $\pm$ 1.116 $\mu$g/animal for the 10-mg/kg test substance VII-2 administered group; 6.991 $\pm$ 0.918 $\mu$g/animal for the 100-mg/kg test substance VII-2 administered group; and 5.665 $\pm$ 0.674 $\mu$g/animal for the 50-mg/kg positive control substance administered group. There was a significant difference between the negative and positive control substance administered groups (P = 0.0157).
In addition, there was a significant difference between the negative control substance administered group and the 100-mg/kg test substance VII-2 administered group (P = 0.0094).

2-2-2 Results of intravenous administration into tail vain Examination (see, Fig. 16(b) )

The mean value (mean) $\pm$ standard deviation (SD) in each of the groups was: 10.816 $\pm$ 2.093 $\mu$g/animal for the negative control substance administered group; and 8.308 $\pm$ 2.050 $\mu$g/animal for the 100-mg/kg test substance VII-2 administered group. It was found, from the results of statistical analysis, that there was no significant difference between the negative control substance administered group and the 100-mg/kg test substance VII-2 administered group, with the mean value being a somewhat lower value for the latter.

3. Discussion

The intraperitoneal administration examination was found to show a significant suppression effect between the 50-mg/kg positive control substance (Hydrocortisone) administered group and the negative control substance administered group (P = 0.0157), and thus would be considered to have no problems with the validity of this examination using the animal model. The 10-mg/kg test substance VII-2 administered group was found to show no significant difference in the suppression effect on the Arthus reaction (P = 0.1765), but to have a difference between the mean values. The 100-mg/kg test substance VII-2 administered group was found to show a significant suppression effect (P = 0.0094). Intravenous administration into tail vain examination, the 100-mg/kg test substance VII-2 administered group was found to show no statistically significant difference in the suppression effect on the Arthus reaction (P = 0.0920). However, the amount of dye leakage in this group, in comparison to the control group, was at a lower value than the mean value in the control group, and was found to show a tendency to suppress the Arthus reaction.
Thus, it was demonstrated that test substance VII-2 exhibited a suppression effect on the type III allergic (Arthus) reaction. Therefore, it was revealed that the peptide of the present invention had a clear and satisfactory suppression effect of immune-complex disease and would be useful in the treatment and prevention of immune complex diseases, such as SLE, glomerulonephritis, arthritis, vasculitis. In addition, it was found, in these examinations, that the peptide of the present invention neither caused any abnormalities nor any adverse effects due to its administration in all the animals tested.

Example 7

Study of Effects of Peptide of The Present Invention on Suppression of IgG Production by Co-Culturing of B Cells with Synovial Cells derived from Rheumatoid Arthritis Patient
It is known that in rheumatic joints, synovial cells and B cells co-exist and stimulate each other, whereby IgG, an inflammatory substance, is produced. Thus, the present inventors examined the effect of a peptide of the present invention on suppression of IgG production using a model system.

1. Experimental Procedures

Synovial cells derived from a rheumatoid arthritis patient (passages 3 to 7) were seeded into 24-well plates (at $3 \times 10^4$ cells/well) and cultured overnight. Test substance VII-2 was added to $10\mu$ g/ml and subjected to pre-treatment for 30 minutes. Then, B cells were added (at $1 \times 10^6$ cells/well) and co-cultured with the synovial cells. After 5 days of culturing, the supernatant was collected to determine the amount of IgG by ELISA (using the Human IgG EIA Kit (TaKaRa, #MK136)). A system which was treated similarly without added test substance VII-2 was used as control.

2. Results

The results are shown in Fig. 17. Test substance VII-2 was demonstrated to suppress the production of IgG down to about three fourths of the control, even at a low concentration of 10 $\mu$g/ml. Further, it was demonstrated that the peptide of the present invention was effective in suppressing activation not only of synovial cells, but also of B cell lines. These results indicate that test substance VII-2 can suppress inflammation also in actual rheumatoid arthritis, and are not inconsistent with the results obtained in the above Examples.

Industrial Applicability

According to the present invention, it is made possible to provide a pharmaceutical composition which is more useful in the treatment or prevention of a disease caused by binding between Clq and an immunoglobulin. It is also made possible to provide a pharmaceutical composition of which the virtual dosage amount is increased in comparison to conventional pharmaceutical compositions, by using in the above-described pharmaceutical composition the peptide of the present invention which has improved water solubility. The pharmaceutical compositions provided by the present invention are effective particularly in treating arthritis and rheumatism. Therefore, the present invention can be utilized, for example, in the fields of pharmaceuticals and research reagents.

Sequence Listing Free Text

SEQ ID NO:1: Immunoglobulin binding peptide
SEQ ID NO:2: Immunoglobulin binding peptide
SEQ ID NO:3: Immunoglobulin binding peptide
SEQ ID NO:4: Immunoglobulin binding peptide
SEQ ID NO:5: Immunoglobulin binding peptide
SEQ ID NO:6: Immunoglobulin binding peptide
SEQ ID NO:7: Immunoglobulin binding peptide
SEQ ID NO:8: Immunoglobulin binding peptide
SEQ ID NO:9: Immunoglobulin binding peptide
SEQ ID NO:10: Immunoglobulin binding peptide
SEQ ID NO:11: Immunoglobulin binding peptide
SEQ ID NO:12: Immunoglobulin binding peptide
SEQ ID NO:13: Immunoglobulin binding peptide
SEQ ID NO:14: Immunoglobulin binding peptide
SEQ ID NO:15: Immunoglobulin binding peptide
SEQ ID NO:16: Nucleic acid encoding immunoglobulin, b inding peptide
SEQ ID NO:17: Nucleic acid encoding immunoglobulin b inding peptide
SEQ ID NO:18: Nucleic acid encoding immunoglobulin b inding peptide
SEQ ID NO:19: Nucleic acid encoding immunoglobulin b inding peptide
SEQ ID NO:20: Nucleic acid encoding immunoglobulin b inding peptide
SEQ ID NO:21: Nucleic acid encoding immunoglobulin b inding peptide
SEQ ID NO:22: Nucleic acid encoding immunoglobulin b inding peptide

**Claims**

1. A peptide capable of binding to an immunoglobulin, having an amino acid sequence represented by:

   Pro-Gly-Leu-$X_4$-$X_5$-Phe

   wherein $X_4$ and $X_5$ are not Tyr at the same time.

2. The peptide according to claim 1, wherein both $X_4$ and $X_5$ are hydrophilic amino acids.

3. The peptide according to claim 1, wherein each of $X_4$ and $X_5$ independently is an amino acid selected from the group consisting of Lys, Arg, His, Asp, Glu, Asn, Gln, Ser, and Thr.

4. The peptide according to claim 1, which has the amino acid sequence depicted in any of SEQ ID NOs. 9 to 15.

5. The peptide according to any of claims 1 to 4, wherein one or more amino acids in the amino acid sequence represented by Formula (I), except for Gly, are substituted by the corresponding D-amino acid(s).

6. The peptide according to any of claims 1 to 4, wherein all of the amino acids in the amino acid sequence represented by Formula (I), except for Gly, are substituted by the corresponding D-amino acids.

7. A fusion protein in which the peptide according to any of claims 1 to 6 is added at the N-and/or C-terminus of a given protein.

8. A nucleic acid encoding the peptide according to any of claims 1 to 4.

9. A nucleic acid encoding a fusion protein in which the peptide according to any of claims 1 to 4 is added at the N-and/or C-terminus.

10. A pharmaceutical composition for the treatment or prevention of a disease caused by binding between C1q and an immunoglobulin, comprising, as the active ingredient, the peptide according to any of claims 1 to 6, the fusion protein according to claim 7, the nucleic acid according to claim 8 or 9, or a vector comprising the nucleic acid according to claim 8 or 9.

11. The pharmaceutical composition according to claim 10, wherein the disease is rheumatoid arthritis.

12. The pharmaceutical composition according to claim 10, wherein the disease is selected from the group consisting of systemic lupus erythematosus (SLE), glomerulonephritis, vasculitis, and arthritis.

13. The pharmaceutical composition according to any of claims 10 to 12, wherein the active ingredient is the peptide according to any of claims 4 to 6.

Fig 1

Fig 2

Fig 3

(A) IL-1b

(B) MMP-1

(C) MMP-3

(D) TNF-α

Fig 4

Test Substance a

Days after administration of monoclonal antibody cocktail

Fig 5

Test Substance α

Days after administration of monoclonal antibody cocktail

Fig 6

Test Substance VII-1

Fig 7

Test Substance VII-2

Fig 8

Test Substance a

Days after administration of monoclonal antibody cocktail

Fig 9

Test Substance α

Days after administration of monoclonal antibody cocktail

Fig 10

Fig 11

Fig 12

Days after administration of monoclonal antibody cocktail

Fig 13

Days after administration of monoclonal antibody cocktail

Fig 14

Days after administration of monoclonal antibody cocktail

Fig 15

Days after administration of monoclonal antibody cocktail

Fig 16

a)

Each value is mean ± S.D.
** :P<0.01 vs Negative control substance
* :P<0.05 vs Negative control substance
n = 5

b)

Fig 17

<table>
<tr><td colspan="2">**INTERNATIONAL SEARCH REPORT**</td><td>International application No.<br>PCT/JP2011/071671</td></tr>
</table>

| A. CLASSIFICATION OF SUBJECT MATTER |
|---|
| *C07K7/06*(2006.01)i, *A61K38/00*(2006.01)i, *A61P9/14*(2006.01)i, *A61P13/12*(2006.01)i, *A61P19/02*(2006.01)i, *A61P29/00*(2006.01)i, *C07K19/00*(2006.01)i, *C12N15/09*(2006.01)i |
| According to International Patent Classification (IPC) or to both national classification and IPC |

| B. FIELDS SEARCHED |
|---|
| Minimum documentation searched (classification system followed by classification symbols)<br>C07K7/06, A61K38/00, A61P9/14, A61P13/12, A61P19/02, A61P29/00, C07K19/00, C12N15/09 |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN), JSTPlus/JMEDPlus/JST7580(JDreamII), WPI

### C. DOCUMENTS CONSIDERED TO BE RELEVANT

| Category* | Citation of document, with indication, where appropriate, of the relevant passages | Relevant to claim No. |
|---|---|---|
| X/Y | WO 2010/084851 A1 (MMT Co., Ltd.),<br>29 July 2010 (29.07.2010),<br>& JP 4705694 B | 1,5-13/2-4 |
| X/Y/A | WO 2009/025300 A1 (Regenetiss Inc., MMT Co., Ltd.),<br>26 February 2009 (26.02.2009),<br>& JP 4313433 B          & US 2010/0167348 A1<br>& EP 2182003 A1 | 1,7-12/2-4/<br>5,6,13 |
| Y | JP 2009-508505 A (Biocompatibles UK Ltd.),<br>05 March 2009 (05.03.2009),<br>& US 2011/0130329 A          & EP 1767545 A1<br>& WO 2007/039140 A1 | 2-4 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>31 October, 2011 (31.10.11) | Date of mailing of the international search report<br>08 November, 2011 (08.11.11) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

| International application No. |
|---|
| PCT/JP2011/071671 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2003-502033 A  (ICOS Corp.),<br>21 January 2003 (21.01.2003),<br>& US 6599727 B1          & EP 1192258 A<br>& WO 2000/077179 A2 | 2-4 |
| Y | Atsushi KATO et al., "Methods for Enhancing<br>Protein Solubility and the Effect of SEP-Tags<br>on Protein Solubility", Biophysics, 2008,<br>vol.48, no.3, pages 185 to 189 | 2-4 |
| A | WO 2008/035527 A1  (MMT Co., Ltd.),<br>27 March 2008 (27.03.2008),<br>& JP 2008-100986 A | 1-13 |
| A | WO 2003/022992 A2  (SCHERING CORP.),<br>20 March 2003 (20.03.2003),<br>& US 2003/0134328 A1 | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009025300 A

- WO 2010084851 A

### Non-patent literature cited in the description

- **OCHI T et al.** *Arthritis Rheum.,* January 1998, vol. 31 (1), 37-43
- **J. SAMBROOK et al.** Molecular Cloning: A Laboratory Manual. Cold Spring Harbor Laboratory Press, 1989

- **FREDERICK M. AUSUBEL et al.** Current Protocols in Molecular Biology. John Wiley & Sons, 1987